# EUROPEAN PATENT APPLICATION

(11) **EP 4 553 083 A1**
(43) Date of publication of application: **14.05.2025**
(21) Application number: 23208968.0
(22) Date of filing: 10.11.2023
(51) Int. Cl.: C07K 14/47, A61K 38/00, A61K 39/00, A61P 25/00, A61P 25/28, A61P 37/06, G01N 33/564

(54) **PEPTIDES AND THEIR USE FOR THE TREATMENT AND/OR PREVENTION OF MULTIPLE SCLEROSIS**

(71) Applicant: Cellerys AG, 8952 Schlieren (CH); Universität Zürich, 8006 Zürich (CH)
(72) Inventor: Martin, Roland, 8952 Schlieren (CH); Lutterotti, Andreas, 8952 Schlieren (CH); Sospedra, Mireia, 8952 Schlieren (CH); Naghavian, Reza, 8952 Schlieren (CH)
(74) Representative: Hoffmann Eitle

(57) **Abstract**

The disclosure relates to novel TSTA-3 and/or RASGRP2 peptides and a combination and a polypeptide or protein comprising such peptides. The peptides or polypeptides or proteins can particularly be used for the treatment and/or prevention of multiple sclerosis (MS), such as in a tolerization approach.

## Description

### FIELD OF INVENTION

The disclosure relates to peptides, combinations thereof and polypeptides or proteins comprising such peptides or combinations. The peptides can particularly be used for the treatment and/or prevention of multiple sclerosis (MS), such as in a tolerization approach.

### BACKGROUND OF THE INVENTION

Multiple sclerosis (MS) is a devastating autoimmune inflammatory disease mainly affecting young adults. MS is a prototypic example of an organ-specific autoimmune disease (AID), as the autoimmune response only targets the central nervous system (CNS) consisting of brain and spinal cord.

During the last thirty years, several myelin proteins such as myelin basic protein (MBP), proteolipid protein (PLP) and myelin oligodendrocyte glycoprotein (MOG) have been identified as encephalitogenic in animal models (experimental autoimmune encephalomyelitis; EAE) (Sospedra and Martin, 2005, Annu Rev Immunol, 23:683-747). The above autoantigens are CNS-specific and almost exclusively expressed in the brain.

Detailed investigation of the immune response against the CNS-specific proteins showed that certain peptides thereof are recognized by a large fraction of patients and in the context of the disease-associated HLA-DR molecules. Such peptides are referred to as immunodominant (Bielekova et al., 2004, J Immunol, 172:3893-3904).

Recently, a novel approach for identifying MS-relevant antigens led to the identification of the autoantigens GDP-L-fucose synthase (gene abbreviation: TSTA3; also known as: GDP-L-fucose:NADP+ 4-oxidoreductase (3,5-epimerizing) or GDP-4-keto-6-deoxy-D-mannose-3,5-epimerase-4-reductase) and proteins of the RASGRP (RAS guanyl releasing protein) family, including RASGRP1, RASGRP2, RASGRP3 and RASGRP4 as well as splice variants and isoforms thereof as particularly relevant in MS (EP 3 813 865, which is incorporated by reference in its entirety). The novel approach included examination of T cells clonally expanded in the brain of an MS patient (homozygous for the HLA DR15 haplotype, which is known to be the major genetic risk factor in MS), who died of a very aggressive form of MS. Thereby, for the first time T cells originating from and clonally expanded in the target organ were analyzed for the purpose of antigen identification. The clonal expansion of a T cell clone (TCC) in MS brain lesions suggests that the cells are MS-relevant. Two T cell clones of the same patient that have been identified as disease-relevant have been analyzed for antigen identification. Target epitopes that are recognized by biologically relevant (e.g. tissue-infiltrating) T cells can thereby be identified. EP 3 813 865 also revealed immunodominant peptides from the identified autoantigens.

Immunodominant peptides can be used in antigen-specific immunotherapies such as tolerance induction. One example is EP 2 205 273 B1, which is incorporated by reference in its entirety, and which discloses immunodominant peptides of MBP, PLP and MOG and their application for MS treatment. In the approach disclosed therein, the peptides are coupled to white or red blood cells.

Currently approved therapies for MS involve various antigen-nonspecific immunomodulating or immunosuppressive strategies, which are only partially effective. All current therapeutics need to be taken orally daily or injected/infused at various time intervals and for long periods of time. Further, they are associated with numerous and sometimes severe side effects.

A therapy that addresses the pathogenesis of MS at its roots should aim to specifically delete or functionally inhibit pathogenic autoreactive cells without altering the "normal" immune system. This is of importance because global immunomodulation and/or immunosuppression come at the cost of inhibiting beneficial regulatory cells and immune cells that serve protective functions against pathogens. Ideally, peptide-specific immune tolerance, that is the specific correction of the misdirected autoimmune response against brain/spinal cord tissue, should be achieved early in the inflammatory phase of the disease, when blockade of the autoreactive immune response can inhibit dissemination and propagation of the disease and irreversible disability can be prevented. Therefore, the preferred targeted patient group are relapsing-remitting MS patients early in the disease course or even patients presenting with a first clinical event suggestive of MS, i.e. clinically isolated syndrome (CIS), or patients, in whom the disease is discovered even earlier at the stage of radiologically isolated syndrome (RIS). At this time point, MS patients generally have a low grade of neurologic disability, which allows them to participate in all activities of daily life and work without significant compromise.

A particular challenge is to identify peptides or combinations of peptides or polypeptides or proteins that can be used in a physiological solution, in particular for the treatment and/or prevention of MS, in particular for the induction of tolerance to MS. For the purpose of tolerance induction, it is advantageous that the peptides or polypeptides or proteins can be coupled to a cell, in particular to a red or white blood cell.

### SUMMARY OF THE INVENTION

It is therefore an object of the present invention to identify peptides or combinations of peptides or polypeptides or proteins based on the newly identified autoantigens TSTA-3 and RASGRP2 that can be used in a physiological solution. A physiological solution comprising the peptide/s and/or a combination comprising the peptides and/or polypeptides or proteins could advantageously be used in a tolerization approach, in particular for the treatment and/or prevention of MS. In such a tolerization approach, for example, a physiological solution comprising the peptides and/or combination comprising the peptides and/or polypeptides or proteins can be coupled to a cell, for example a red or white blood cell.

It was surprisingly found that a peptide comprising or consisting of the sequence of TSTA3-3 (242-251) (SEQ ID NO: 1) and/or a peptide comprising or consisting of the sequence of RASGRP2 (74-91) (SEQ ID NO: 2) and/or a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1 and/or SEQ ID NO: 2, respectively, wherein the peptide is soluble in a physiological solution and has a maximum length of 50 amino acids can be used in a physiological solution, for example for the treatment and/or prevention of MS, in particular for inducing tolerance to autoantigens in MS.

In particular for the purpose of coupling the peptides or polypeptides or proteins to a cell for a tolerization approach, solubility of the peptides or polypeptides or proteins in a physiological solution is advantageous. It has been presently shown that the inventive soluble peptides can be efficiently coupled to a cell. It has also been exemplarily shown that peptide RASGRP2 (74-91) (SEQ ID NO: 2) can induce IFN-γ secretion and proliferation of T cells. In the examples shown, biotinylation as a possible marker of a peptide does not interfere with these biological functions of the peptide.

Biodistribution assays showed that peptide-coupled red blood cells efficiently target liver and spleen of mice and humans.

Hence, the identified peptides are very suitable for the treatment, prevention and/or diagnosis, preferably *in vitro* diagnosis, of MS, for example for inducing antigen-specific tolerance to autoantigens in a human subject suffering from or at risk of developing MS.

A peptide comprising or consisting of the sequence of RASGRP2 (74-91) (SEQ ID NO: 2) and/or a peptide comprising or consisting of a sequence with at least 80 %, preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 2, wherein the peptide has a maximum length of 50 amino acids, and a peptide consisting of the sequence of TSTA3-3 (242-251) (SEQ ID NO: 1) or RASGRP2 (74-91) (SEQ ID NO: 2) is also provided.

In another aspect of the invention, a combination comprising one or more of the above peptides and one or more of the following peptides is provided:
a) a peptide comprising or consisting of the sequence of MBP1 (13-32) (SEQ ID NO: 3),
b) a peptide comprising or consisting of the sequence of MBP2 (83-99) (SEQ ID NO: 4),
c) a peptide comprising or consisting of the sequence of MBP3 (111-129) (SEQ ID NO: 5),
d) a peptide comprising or consisting of the sequence of MBP4 (146-170) (SEQ ID NO: 6),
e) a peptide comprising or consisting of the sequence of MOG1 (1-20) (SEQ ID NO: 7),
f) a peptide comprising or consisting of the sequence of MOG2 (35-55) (SEQ ID NO: 8),
g) a peptide comprising or consisting of the sequence of PLP1 (139-154) (SEQ ID NO:9),
h) a peptide comprising or consisting of the sequence of TSTA3-1 (51-65) (SEQ ID NO: 10),
i) a peptide comprising or consisting of the sequence of TSTA3-2 (136-150) (SEQ ID NO: 11),
j) a peptide comprising or consisting of the sequence of TSTA3-4 (296-310) (SEQ ID NO: 12),
k) a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or SEQ ID NO: 12, respectively.

The combination according to the invention may be used in a tailored treatment approach depending on the specific patient. The combination can for example be adapted depending on the specificity of the T cells of each individual patient.

In a further aspect of the invention, a combination comprising or consisting of the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12) is provided.

This particular combination comprises peptides that are particularly advantageous because they have been shown to be immunodominant in MS patients. The combination is therefore particularly suitable for the treatment and/or prevention of MS, for example in a tolerization approach when coupled to cells, in particular to red or white blood cells.

In a further aspect of the invention, a polypeptide or protein comprising at least two of the above peptide sequences is provided, optionally comprising a linker, preferably GGS, between at least two of the peptide sequences.

This polypeptide or protein is advantageous because using such a polypeptide or protein, e.g. for the treatment and/or prevention of MS, preferably ensures that all of the peptides that are comprised within the polypeptide or protein are used in definite amounts. For example, if a polypeptide or protein comprises six of above peptides, i.e. for example six peptides are linked to one another to result in a polypeptide or protein, in a tolerization approach using such polypeptide or protein it is preferably ensured that all six peptides are used and one of each in each polypeptide or protein.

In a particular embodiment, at least one of the peptides and/or at least one, preferably all peptides of the combination, and/or at least one polypeptide or protein is immunodominant or all peptides and/or polypeptides or proteins are immunodominant. Immunodominance for example suggests that the peptides are more likely to be recognized by a patient's T cells.

In a preferred embodiment, at least one peptide and/or polypeptide or protein is coupled with a marker. A marker can for example be biotin or a fluorescent dye, such as FAM or Cy7; preferably the marker is biotin. A marker advantageously allows detection of the coupling reaction as shown in the present examples. For example, the peptide RASGRP2 (74-91) (SEQ ID NO: 2) is biotinylated.

One other aspect of the invention relates to a carrier, preferably a cell or a population of cells, chemically coupled to at least one of the peptides and/or combinations and/or polypeptides or proteins as disclosed *supra.* A method of manufacturing the chemically coupled cell or population of cells is also provided comprising providing an isolated cell, preferably a blood cell, preferably an RBC, or a population of isolated cells, preferably a population of blood cells, preferably a population of RBCs, from a human subject, adding the peptide and/or the combination and/or the polypeptide or protein of the present invention and subsequently adding a coupling agent, preferably 1-ethyl-3-(3-dimethylaminopropyl)-carbodiimide (ECDI/EDC). In one embodiment, the cell is a red or white blood cell, preferably a red blood cell. Efficient coupling of the inventive peptides has presently been shown as disclosed *infra.*

It has also been presently shown that after coupling of a RASGRP2 peptide (SEQ ID NO: 2; which had been biotinylated) to a red blood cell, and incubation of the coupled red blood cells with active monocytes, the monocytes are able to engulf, process and present the antigen on their surface. This is a prerequisite in the process of tolerization.

Further, a T cell clone recognizing RASGRP2 has been surprisingly shown to secrete IL-10 upon cultivation with monocytes presenting RASGRP2 peptide or in response to soluble RASGRP2. This tolerogenic response has been shown to be antigen-specific. In addition, it has been shown that CD4+ T cells appear to be responsible for the IL-10 (and IFN-γ) production. The secretion of IL-10 has not been observed with the T cell clone upon presentation of the peptide with other antigen-presenting cells.

The results surprisingly indicate that autoreactive and proinflammatory T cells can be functionally reprogrammed upon presentation of antigens by antigen-presenting cells that have engulfed and processed peptide-coupled red blood cells, essentially allowing an efficient tolerization.

### BRIEF DESCRIPTION OF THE FIGURES

- Figure 1: Influence of peptide concentration on coupling efficiency
- Figure 2: Influence of EDC concentration on coupling efficiency
- Figure 3: Coupling efficiency of 12 peptides
- Figure 4: Coupling efficiency (biotinylation at the side chain vs. the N-terminus)
- Figure 5: T cell reactivity in PBMCs in response to different peptides (IFN-γ fluorospot assay)
- Figure 6: T cell reactivity in RASGRP2 (78-87) specific T cell clones (3H-thymidine assay)
- Figure 7: *In-vivo* and *ex-vivo* biodistribution of dye-coupled red blood cells in mice
- Figure 8: *Ex-vivo* histological analysis of organs
- Figure 9: *In-vivo* biodistribution of peptide-coupled RBCs in MS patients
- Figure 10: CD169 expression after stimulation of monocytes with IFN-α (24 h and 48 h)
- Figure 11: CFSE labeling of red blood cells (RBCs) and RBCs coupled to biotinylated RASGRP2 peptide
- Figure 12: Presentation of peptide by CD169+ monocytes
- Figure 13: Plate setup for experiment on secretion of cytokines upon co-cultivation of TCC14, monocytes and peptide-coupled RBCs
- Figure 14: Secretion of IL-10 and IFN-γ upon co-cultivation of TCC14, monocytes and peptide-coupled RBCs (IFN-y/IL-10/IL-17 fluorospot assay)
- Figure 15: Antigen specificity of TCC14
- Figure 16: Intracellular cytokine staining of CD4+ cells

### DETAILED DESCRIPTION OF THE INVENTION

Based on the newly identified autoantigens GDP-L-fucose synthase (TSTA-3) and RASGRP2 in MS (cf. EP 3 813 865), the present study led to the identification of particularly useful peptides of these autoantigens which can for example be used in the treatment and/or prevention of multiple sclerosis (MS). Diagnosis, in particular *in vitro* diagnosis, can also be performed using these peptides. Autoantigen preferably means a self-antigen (as opposed to a foreign antigen) that the immune system of an autoimmune patient recognizes as foreign.

The present invention relates to a peptide comprising or consisting of the sequence of TSTA3-3 (242-251) (SEQ ID NO: 1) and/or a peptide comprising or consisting of the sequence of RASGRP2 (74-91) (SEQ ID NO: 2) and/or a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1 and/or SEQ ID NO: 2, respectively, wherein the peptide is soluble in a physiological solution and has a maximum length of 50 amino acids.

The present invention also relates to a peptide comprising or consisting of the sequence of RASGRP2 (74-91) (SEQ ID NO: 2) and/or a peptide comprising or consisting of a sequence with at least 80 %, preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 2, wherein the peptide has a maximum length of 50 amino acids.

In particular, the present invention relates to a peptide consisting of the sequence of TSTA3-3 (242-251) (SEQ ID NO: 1) or RASGRP2 (74-91) (SEQ ID NO: 2).

The peptide is preferably soluble in a physiological solution. The peptide is preferably used in the treatment and/or prevention of MS.

In one embodiment, at least one peptide of the present invention is used in combination. For example, a combination can comprise 2 or 3 or 4 or 5 or 6 or 7 or 8 or 9 or 10 or 11 or 12 or more different peptides. A peptide is different from another peptide if it does not consist of the same amino acid sequence and/or does not have the same modification.

A combination may for example comprise one or more of the TSTA-3 and/or RASGRP2 peptides as disclosed *supra* and one or more of the myelin peptides, in particular one or more or all of the myelin peptides as defined by SEQ ID NOs: 3 to 9. In a particular embodiment, at least one of the peptides is immunodominant or all peptides are immunodominant.

The present invention also relates to a combination comprising one or more of the TSTA-3 and/or RASGRP2 peptides as disclosed *supra* and one or more of the following peptides:
a) a peptide comprising or consisting of the sequence of MBP1 (13-32) (SEQ ID NO: 3),
b) a peptide comprising or consisting of the sequence of MBP2 (83-99) (SEQ ID NO: 4),
c) a peptide comprising or consisting of the sequence of MBP3 (111-129) (SEQ ID NO: 5),
d) a peptide comprising or consisting of the sequence of MBP4 (146-170) (SEQ ID NO: 6),
e) a peptide comprising or consisting of the sequence of MOG1 (1-20) (SEQ ID NO: 7),
f) a peptide comprising or consisting of the sequence of MOG2 (35-55) (SEQ ID NO: 8),
g) a peptide comprising or consisting of the sequence of PLP1 (139-154) (SEQ ID NO:9),
h) a peptide comprising or consisting of the sequence of TSTA3-1 (51-65) (SEQ ID NO: 10),
i) a peptide comprising or consisting of the sequence of TSTA3-2 (136-150) (SEQ ID NO: 11),
j) a peptide comprising or consisting of the sequence of TSTA3-4 (296-310) (SEQ ID NO: 12), or
k) a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or SEQ ID NO: 12, respectively.

An identity of at least 80 %, more preferably at least 85 %, more preferably of at least 90 %, more preferably at least 95 %, more preferably at least 96 %, more preferably at least 97 %, more preferably at least 98 %, more preferably at least 99 % is especially preferred.

The myelin proteins MBP, PLP and MOG are the main constituents of the myelin sheath, important autoantigens in MS and can also be used for inducing experimental autoimmune encephalomyelitis (EAE), an animal model of MS. It has been shown that the frequency of high avidity CD4+ T cells reactive to MBP, PLP and MOG is increased in MS patients (Bielekova et al., 2004). EP 2 205 273 B1 which is incorporated herein by reference in its entirety discloses immunodominant peptides of MBP, PLP and MOG and their application for MS treatment.

In a preferred embodiment, at least one, preferably all peptides of the combination each has/have a maximum length of 50 amino acids.

In particular, the present invention relates to a combination comprising or consisting of the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12).

The combination according to the present invention may in particular be used in the treatment and/or prevention of MS. In a particularly preferred embodiment, RASGRP2 (74-91) (SEQ ID NO: 2) is biotinylated.

A combination of peptides is particularly useful to tailor a treatment for a specific human subject, i.e. an MS patient. Based on the immune recognition of this specific patient, those peptides that are immunodominant can be preferably chosen for a treatment. The pathogenic autoimmune response can thereby be specifically attenuated and with few side effects. Good safety and tolerability can thus be expected.

The present invention also relates to a polypeptide or protein comprising at least two of any of the above peptide sequences, optionally with a linker, preferably GGS, between at least two of the peptide sequences. Hence, the polypeptide or protein comprises at least one of:
- a peptide comprising or consisting of the sequence of TSTA3-3 (242-251) (SEQ ID NO: 1) and/or
- a peptide comprising or consisting of the sequence of RASGRP2 (74-91) (SEQ ID NO: 2) and/or
- a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1 and/or SEQ ID NO: 2, respectively,
wherein the peptide is soluble in a physiological solution and has a maximum length of 50 amino acids.

Thereby, the peptide sequences for example concatenate into an artificial polypeptide or protein, i.e. the peptide sequences are linked to one another, preferably covalently linked. This polypeptide or protein comprises a definite amount and order of the at least two peptides. Therefore, using this polypeptide or protein, for example in a coupling reaction (coupling the polypeptide or protein to, e.g., a cell), preferably ensures that all of the peptides that are comprised within the polypeptide or protein are coupled. In addition, the subject's immune system preferably cuts the polypeptide or protein into peptides for optimal antigen presentation instead of presenting pre-cut peptides. The cut peptides are preferably able to bind to an autologous HLA allele and/or to be recognized by a T cell. In addition or alternatively, the cut peptides are preferably recognized by an antibody.

In a particular embodiment, the polypeptide or protein comprises 2 or 3 or 4 or 5 or 6 or 7 or 8 or 9 or 10 or 11 or 12 or more different peptide sequences. It is especially preferred to link 6 to 12 peptides. It is also possible to incorporate the same peptide more than once. Particularly preferred polypeptides or proteins are those comprising or consisting of at least one of the sequences as set forth in SEQ ID NOs: 33-37 or those comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with any one of the sequences of SEQ ID NOs: 33 to 37. It is particularly preferred to link the 12 different peptides with SEQ ID NOs: 1-12 into one protein, such as for example in proteins with SEQ ID NO: 33, 34 or 37. A protein comprising or consisting of the sequence as set forth in SEQ ID NO: 37 is especially preferred, preferably a protein consisting of the sequence as set forth in SEQ ID NO: 37.

The order of the peptides within the polypeptide or protein may be adapted based for example on the solubility and efficacy of the polypeptide or protein. A certain order may also be advantageous regarding manufacture, efficiency of expression, secondary structures, interaction with other proteins or cells or binding to carriers. Based on these and other criteria the person skilled in the art can determine a suitable order.

A linker between at least two of the peptide sequences is optional. A linker may comprise one or 2 or 3 or more amino acids. Preferably, the linker comprises 3 or less amino acids. For example, the linker may be VVR and/or GGS. Within the same polypeptide or protein, the same or different linkers can be used. In a particularly preferred embodiment, the linker is GGS.

Preferably, the polypeptide or protein is soluble in a physiological solution. The polypeptide or protein may in particular be used in the treatment and/or prevention of MS.

A peptide is intended to mean two or more amino acids joined together by peptide bonds. A peptide according to the invention preferably has a maximum length of 50 amino acids. In a particular embodiment, a peptide has a maximum length of 30 amino acids, preferably a maximum length of 25 amino acids.

A polypeptide is intended to mean two or more peptides. A protein is intended to mean one or more polypeptides. In one embodiment, a protein comprises 100 or more amino acids. The terms polypeptide and protein are used interchangeably herein. A protein sequence may be defined by a GenBank entry. A protein sequence may also be defined by a UniProtKB/Swiss-Prot entry and/or by a GenPept entry. An entry may be defined by a number, e.g. an accession number. Where applicable, the database entries include the respective accession number (i.e. an entry number) and version number. A protein may also be defined by any other database known to the skilled person.

The peptide and/or at least one peptide of the combination and/or the polypeptide or protein can be functionalized at one end or both ends and/or within the peptide and/or at least one peptide of the combination and/or the polypeptide or protein for easier isolation and/or detection, e.g. by a His Tag. A His Tag preferably comprises two to ten histidine residues. In one embodiment, the His Tag consists of six histidine residues. The His Tag may for example be located at the N-terminus of the peptide and/or polypeptide or protein.

The peptide and/or at least one peptide of the combination and/or the polypeptide or protein can also have other modifications such as one or more N- or C-terminal modifications or modifications at a side chain of an amino acid. Modifications preferably improve properties of the peptide and/or polypeptide or protein, such as stability, solubility and/or the ability to be detected. A preferred modification is a detectable one, for example by FACS. Further, the modification is preferably non-toxic and is safe to use in subjects, in particular human subjects. Further, the modification preferably does not interrupt with the function of the peptide and/or combination and/or polypeptide or protein, in particular
- with the interaction with the HLA molecule and/or T cell receptor, and/or
- coupling of the peptide and/or polypeptide or protein to a carrier, in particular to a cell.

Possible modifications comprise biotinylation, attachment of a fluorescent dye, such as FAM or Cy7, preferably FAM, acetylation, phosphorylation or the like. It has been presently shown that biotinylation can be used to detect the efficient coupling of the peptide to a cell. Biotinylation also did not affect recognition by T cells. In a preferred embodiment, the modification is a biotinylation.

Hence, in one embodiment, the at least one peptide and/or polypeptide or protein is coupled with a marker. Preferably, the marker is biotin or a fluorescent dye, such as FAM or Cy7; preferably the marker is biotin. Two different markers are also possible in the same reaction, for example biotin labeling of one peptide and FAM labeling of another peptide.

Biotinylation can be performed at the N-terminus, C-terminus or within the peptide and/or polypeptide or protein (i.e. at a side chain of an amino acid).

Different amino acid functional groups are available for biotinylation:
a) Primary amines (-NH2): This group is located at the N-terminus of each peptide and/or polypeptide or protein and at the side chain of lysine residues.
b) Sulfhydryls (-SH): This group (sulfhydryl group or thiol group) is located at the side chain of cysteine.
c) Carboxyls (-COOH): This group is located at the C-terminus of each peptide and/or polypeptide or protein and at the side chains of aspartic acid and glutamic acid.
d) Carbonyls (-CHO): This aldehyde group can be created by oxidizing carbohydrate groups in glycoproteins.

Nonselective biotinylation reagents are also available to modify peptides and/or polypeptides or proteins with no available primary amines, sulfhydryls, carboxyls or carbonyls.

In one embodiment, the biotin is indirectly coupled to the peptide and/or polypeptide or protein, for example by a polyethylene glycol (PEG) linker.

Besides affecting the function of the peptide and/or polypeptide or protein, the site of biotinylation may have an influence on coupling efficiency and/or solubility of the peptide and/or polypeptide or protein. Biotinylation at the N-terminus or at a side chain is preferred. Particularly preferred is biotinylation at a side chain of an amino acid of the peptide and/or polypeptide or protein, in particular wherein the amino acid is cysteine. However, other sites can be chosen, in particular when the peptide and/or polypeptide or protein has no cysteine or when the cysteine/s is/are within a region that is necessary for binding to an HLA molecule or T cell receptor.

Preferably, the marker, preferably the biotinylation is within the first 15, preferably within the first 10, more preferably within the first 5 amino acids from the N-terminus and/or within the last 15, preferably within the last 10, more preferably within the last 5 amino acids from the C-terminus. Thereby, the function of the peptide and/or polypeptide or protein including its binding to HLA molecules and recognition by T cells can preferably be preserved. Hence, a preferred site for biotinylation is a cysteine within the first 15, preferably within the first 10, more preferably within the first 5 amino acids from the N-terminus and/or within the last 15, preferably within the last 10, more preferably within the last 5 amino acids from the C-terminus.

All peptides (for example of a combination or polypeptide or protein) can possibly be modified, preferably biotinylated, but preferably only one peptide would be modified, preferably biotinylated, to save costs (for example, when coupled to cells). Any peptide can be chosen. For example, the peptide as defined by any one of SEQ ID NOs: 1-12 is biotinylated. Preferably, RASGRP2 (74-91) (SEQ ID NO: 2) is biotinylated, preferably on the cysteine.

A marker, such as FAM or Cy7, can also be directly coupled to cells, for example to RBCs. Thereby, a cell can be directly detected. If additionally a coupled peptide carries a (e.g. different) marker, cell and peptide can be detected.

An "identity" of an amino acid sequence is preferably determined according to the invention over the entire length of the reference amino acid sequence and refers to identical amino acids. The at least one peptide or combination of peptides or polypeptide or protein with a certain identity as described is preferably immunodominant. In a preferred embodiment, the at least one peptide or combination of peptides or polypeptide or protein can bind to an autologous HLA allele and/or is recognized by a T cell. In addition or alternatively, the at least one peptide or combination of peptides or polypeptide or protein is recognized by an antibody.

A peptide or polypeptide or protein comprising the sequence defined by a SEQ ID NO preferably means that the comprised sequence defined by a SEQ ID NO (alternatively with a given percentage of identity to a SEQ ID NO) may have further amino acids at the N-terminal and/or C-terminal ends. These further amino acids may be any amino acids as long as the peptide is soluble in a physiological solution and has a maximum length of 50 amino acids. The N-terminal and/or C-terminal ends of the peptide may also be similar or identical to the natural surroundings of the peptide in the respective protein. The proteins may be defined by the following SEQ ID NOs referring to full-length human proteins (cf. also Table 1 *infra*):

| | |
|---|---|
| Tissue-specific transplantation antigen P35B (TSTA-3) (also known as: GDP-L-fucose synthase or GDP-L-fucose:NADP+ 4-oxidoreductase (3,5-epimerizing) or GDP-4-keto-6-deoxy-D-mannose-3,5-epimerase-4-reductase) | SEQ ID NO: 13 |
| RAS guanyl releasing protein 2 (RASGRP2) | SEQ ID NOs: 14-18 (different isoforms) |
| myelin basic protein (MBP) | SEQ ID NO: 19 |
| myelin oligodendrocyte glycoprotein (MOG) | SEQ ID NO: 20 |
| proteolipid protein (PLP) | SEQ ID NO: 21 |

The peptides, polypeptides, and proteins, or combinations thereof, of the present invention are preferably isolated peptides, polypeptides, and proteins, or combinations thereof. The term "isolated," as used herein, preferably refers to material that is free to varying degrees from components which normally accompany it as found in its native state. "Isolate" in particular denotes a degree of separation from original source or surroundings. The term "isolated" also embraces recombinant nucleic acids or peptides, as well as chemically synthesized nucleic acids or peptides. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

By an "isolated peptide," "isolated protein," or "isolated polypeptide" is preferably meant a peptide, protein or polypeptide of the invention that has been separated from components that naturally accompany it. Typically, the peptide, protein or polypeptide is isolated when it is at least about 40%, at least about 50%, at least about 60%, at least about 70%, at least about 80%, at least about 90%, and at least about 99%, by weight, free from the proteins and naturally-occurring organic molecules with which it is naturally associated. An isolated polypeptide may be obtained, for example, by extraction from a natural source; by expression of a recombinant nucleic acid encoding such a polypeptide; or by chemically synthesizing the polypeptide. Purity can be measured by any standard, appropriate method, for example, column chromatography, polyacrylamide gel electrophoresis, or by HPLC analysis.

In one embodiment, the peptide and/or polypeptide or protein and/or the nucleic acid (encoding the peptide and/or polypeptide or protein) is isolated.

Peptide or protein solubility is an important issue in the pharmaceutical industry because insolubility of a potentially effective peptide or protein drug leads to unsuitability for preclinical or clinical development.

Solubility may be defined as the ability of a substance, for example a peptide, polypeptide or protein, to form a solution with the solvent, for example saline. More specifically, peptide or protein solubility can be defined as the concentration of soluble peptide or protein that is in equilibrium with a crystalline solid phase under given conditions. Peptide or protein solubility can for example be influenced by the extrinsic factors ionic composition, ionic strength, temperature and pH. Physicochemical properties of the peptide or protein (e.g. ionisation or crystallinity) and also the length of the peptide or protein may also influence solubility.

Solubility of peptides or proteins may also be dependent on the amino acid composition of the peptide or protein. For example, a high content of hydrophobic amino acids may be considered disadvantageous for solubility. A high content of polar amino acids is generally considered advantageous. In general, the pH at which the net electric charge on a peptide or protein is zero is known as the isoelectric point (pl) of the peptide or protein, and this peptide or protein which has an equal number of positive and negative charges is known as a zwitterion. If the pl of the peptide or protein is close to the pH of the buffer (i.e. the solvent), solubility is usually poor. It is usually advantageous if the pH of the buffer is further away from the pl of the peptide or protein, for example at least 2 units away from the pl of the peptide or protein. However, solubility in a particular condition is hardly predictable.

Solubility can for example be expressed as grams of solute per 100 milliliters of solvent (g/(100 mL)) at a specific temperature, for example room temperature. Room temperature may for example be between 20°C and 25°C, preferably 24-25°C. Alternatively, the solubility of a solute can be expressed in moles instead of mass, for example mol/kg if the quantity of solvent is given in kg.

A solved peptide or protein in a solvent, for example a physiological solution, preferably leads to a transparent, particle-free solution.

A physiological solution, a preferable solvent according to the present invention, is a diluent that may be used to maintain integrity and viability of cells. A physiological solution is usually isotonic or close to being isotonic. Saline, a mixture of sodium chloride (usually 0.9 %, usually pH between 5 and 6, preferably 5.5), can for example be considered a physiological solution. Ringer's lactate solution can also be used. Preferably, saline is used as a physiological solution.

Solubility can for example be measured by a kinetic solubility assay or a thermodynamic solubility assay (Sou and Bergström, 2018, CAS; Drug Discovery Today: Technologies, 27:11-19). The kinetic (turbidimetric) solubility of a compound can be defined as the maximum solubility of the fastest precipitating species of the compound. The test protocol may for example include that solid compounds are first dissolved in DMSO and then linear serial dilutions of each compound are added to an aqueous buffer and observed for precipitate formation when the compound is not completely soluble. Precipitate appearance can be evaluated by light scattering (laser nephelometry method). For better precision, the solution can be subjected to high-speed centrifugation or filtration using special solubility filter plates and then the compound concentration is measured in the saturated solution directly by UV or LC-MS using separately built calibration curves. The thermodynamic (equilibrium) solubility is the saturation solubility of a compound at the end of the dissolution process, where the dissolved compound is in equilibrium with the undissolved material in excess. In this assay, usually a solvent of choice, for example a physiological solution, for example saline, can be used. Solubility can also be tested by solving the peptide or protein in a solvent at different concentrations and analyzing whether the result is a cloudy solution with precipitation present (insoluble) or whether the result is a clear solution (soluble). "Cloudy" may also be described as "milky". "Cloudy" or "clear" can be determined by visual inspection. For example, one can make use of the "Tyndall effect" when inspecting a liquid. A light beam directed at the liquid will not be visible in a solution but visible in a colloid or suspension. "Cloudy" or "clear" can also be verified by automated processes, such as UV absorption, allowing an objective assessment, for example by comparing with reference solutions. Assays for measuring the solubility are well-known to the person skilled in the art.

Solubility is in particular advantageous in a tolerization approach: after manufacturing, the peptides or polypeptides or proteins are advantageously resuspended in saline. In a preferred embodiment, the peptides and/or polypeptides and/or proteins are then coupled to red blood cells. Before infusion, the coupled cells are again preferably resuspended in saline. Hence, not only the peptides or polypeptides or proteins but also the preferred drug product, the coupled cells, preferably the coupled red blood cells, are preferably soluble in a physiological solution such as saline.

The binding to an autologous HLA allele, recognition by a T cell and/or recognition by an antibody may indicate immunodominance of a peptide, polypeptide or protein. The following characteristics for example indicate that a certain peptide (this term also being used for polypeptides or proteins) is immunodominant in the context of MS:
a) frequent recognition of this peptide by T cells, i.e. by approximately 10% or more of MS patients, often in the context of a disease-associated HLA allele or haplotype (Sospedra and Martin, 2005), and
b) recognition of this peptide by disease-relevant T cells such as those that respond to peptides at low concentrations (high avidity T cells) (Bielekova et al., 2004) and are therefore considered particularly dangerous, and/or have a proinflammatory phenotype, and/or are isolated from the target organ or compartment (CNS), in the case of MS, brain-, spinal cord- or CSF-infiltrating T cells.

However, high avidity recognition is not a prerequisite, since low-avidity myelin-specific T cells have also been shown to be pathogenic in humanized transgenic mouse models (Quandt et al., 2012, J Immunol, 189(6):2897-2908).

Thus, it can be tested whether a peptide, polypeptide or protein is immunodominant in the context of MS. Such a test is preferably an *in vitro* or *ex vivo* test. Particularly suitable is an *in vitro* test that allows measuring the reactivity of T cells, preferably CSF-infiltrating CD4⁺ T cells, and/or antibodies against the peptide and/or the combination and/or the polypeptide or protein, which T cells and/or antibodies have been previously obtained from blood, CSF or other body fluid of the subject. In one embodiment, the subject had previously been diagnosed with MS. In another embodiment, the test is being used for diagnosing MS, in particular for diagnosing pattern I and/or pattern II MS.

The person skilled in the art is aware of methods testing the reactivity of T cells, preferably CD4⁺ T cells, and/or antibodies. For example, the proliferation of T cells, preferably CD4⁺ T cells, (for example by measuring incorporated radioactivity) and/or their secretion of IFN-γ or reactivity in a ELISPOT/FLUOROSPOT assay or reactivity against HLA-peptide tetramers can be tested. If the tested protein or peptide induces reactivity in a human subject that had been diagnosed with MS, in case of T cell reactivity in particular a stimulatory index (SI) above 2 and/or an IFN-γ secretion above 20 pg/ml, the tested protein or peptide may be termed immunodominant. It is also possible to select 10 patients who had been diagnosed with MS for such a test. If reactivity is induced in at least 2 patients, the tested protein or peptide may be termed immunodominant. Preferably, the 10 patients have been diagnosed with RRMS according to the established revised McDonald criteria.

It has recently been demonstrated that T cells of MS patients show increased *in vitro* proliferation in the absence of an exogenous antigen (Mohme et al., 2013, Brain, 136:1783-1798; Jelcic et al., 2018, Cell, 175(1):85-100.e23). These "autoproliferating" T cells are enriched for cells that home to the CNS compartment of MS patients and can thus be considered as a peripheral blood source of brain-/CSF-infiltrating T cells.

Binding of a peptide and/or a protein to an antibody can be measured by standard assays known to the person skilled in the art, for example by ELISA.

In the case that data from testing T cells *in vitro* or ex *vivo* is not available or in addition to such testing, immune recognition of peptides can also be predicted/inferred from those peptides that will bind well to the HLA-class I or -class II alleles of the individual and for CD8+ and CD4+ T cells respectively. Peptide binding predictions are well known to the skilled person. They can be performed by well-established in silico peptide binding prediction algorithms (NetMHCII - www.cbs.dtu.dk/services/NetMHCII/; IEDB - www.iedb.org/) and analysis of the HLA-binding motifs (SYFPEITHI - www.syfpeithi.de/).

It is not necessary that the binding to the HLA allele is particularly strong. In fact, immunodominance can also occur for peptides that bind poorly to the HLA allele (Muraro et al., 1997, J Clin Invest, 100(2):339-349).

In the present invention, a peptide comprising or consisting of the sequence of TSTA3-3 (242-251) (SEQ ID NO: 1), a peptide comprising or consisting of the sequence of RASGRP2 (74-91) (SEQ ID NO: 2), a peptide comprising or consisting of the sequence of MBP1 (13-32) (SEQ ID NO: 3), a peptide comprising or consisting of the sequence of MBP2 (83-99) (SEQ ID NO: 4), a peptide comprising or consisting of the sequence of MBP3 (111-129) (SEQ ID NO: 5), a peptide comprising or consisting of the sequence of MBP4 (146-170) (SEQ ID NO: 6), a peptide comprising or consisting of the sequence of MOG1 (1-20) (SEQ ID NO: 7), a peptide comprising or consisting of the sequence of MOG2 (35-55) (SEQ ID NO: 8), a peptide comprising or consisting of the sequence of PLP1 (139-154) (SEQ ID NO:9), a peptide comprising or consisting of the sequence of TSTA3-1 (51-65) (SEQ ID NO: 10), a peptide comprising or consisting of the sequence of TSTA3-2 (136-150) (SEQ ID NO: 11), a peptide comprising or consisting of the sequence of TSTA3-4 (296-310) (SEQ ID NO: 12), and/or a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1, 2, 3, 4, 5, 6, 7, 8, 9, 10, 11 and/or SEQ ID NO: 12, respectively, wherein the peptide is soluble in a physiological solution and has a maximum length of 50 amino acids have been shown to be immunodominant. In particular, the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12) have been shown to be immunodominant.

The sequences of SEQ ID NO: 1-32 are listed in the following Table 1:

**Table 1: Peptide and protein sequences, position and database entry. The sequences are human sequences. The sequence Q7LDG7-1 is the canonical sequence of the RASGRP2 protein and also refers to RASGRP2 Isoform 1. Proteins and peptides depicted hereunder are particularly preferred embodiments of the invention.**

| SEQ ID NO: | Sequence (amino acids) | Position | Database |
|---|---|---|---|
| 1 | LSVGEEDEVS | 242-251 | GenBank AAH93061.1 (GDP L-fucose synthase) |
| 2 | KTCHLVRYWISAFPAEFD | 127-144 | GenBank AAI10307.1 (RASGRP2) |
| | | or 74-91 | or |
| | | | UniProtKB/Swiss -Prot Q7LDG7-1 (RASGRP2 Isoform 1) |
| 3 | KYLATASTMDHARHGFLPRH | 13-32 | UniProtKB/Swiss -Prot P02686-5 (MBP Isoform 5) (first amino acid not counted for numbering) |
| 4 | ENPVVHFFKNIVTPRTP | 83-99 | UniProtKB/Swiss -Prot P02686-5 (MBP Isoform 5) |
| | | | (first amino acid not counted for numbering) |
| 5 | LSRFSWGAEGQRPGFGYGG | 111-129 | UniProtKB/Swiss -Prot P02686-5 (MBP Isoform 5) (first amino acid not counted for numbering) |
| 6 | AQGTLSKIFKLGGRDSRSGSPMARR | 146-170 | UniProtKB/Swiss -Prot P02686-5 (MBP Isoform 5) (first amino acid not counted for numbering) |
| 7 | GQFRVIGPRHPIRALVGDEV | 1-20 | UniProtKB/Swiss -Prot Q16653-1 (MOG Isoform 1) (first 29 amino acids not counted for numbering) |
| 8 | MEVGWYRPPFSRVVHLYRNGK | 35-55 | UniProtKB/Swiss -Prot Q16653-1 (MOG Isoform 1) (first 29 amino acids not counted for numbering) |
| 9 | HCLGKWLGHPDKFVGI | 139-154 | UniProtKB/Swiss -Prot P60201-1 (PLP Isoform 1) (first amino acid not counted for numbering) |
| 10 | TAQTRALFEKVQPTH | 51-65 | GenBank AAH93061.1 (GDP L-fucose synthase) |
| 11 | PHNSNFGYSYAKRMI | 136-150 | GenBank AAH93061.1 (GDP L-fucose synthase) |
| 12 | FRFTPFKQAVKETCA | 296-310 | GenBank AAH93061.1 (GDP L-fucose synthase) |
| 13 | MGEPQGSMRILVTGGSGLVGKAIQKVVADGAGL PGEDWVFVSSKDADLTDTAQTRALFEKVQPTHVI HLAAMVGGLFRNIKYNLDFWRKNVHMNDNVLHS AFEVGARKVVSCLSTCIFPDKTTYPIDETMIHNGP PHNSNFGYSYAKRMIDVQNRAYFQQYGCTFTAVI PTNVFGPHDNFNIEDGHVLPGLIHKVHLAKSSGS ALTVWGTGNPRRQFIYSLDLAQLFIWVLREYNEV EPIILSVGEEDEVSIKEAAEAVVEAMDFHGEVTFD TTKSDGQFKKTASNSKLRTYLPDFRFTPFKQAVK ETCAWFTDNYEQARK | 1-321 (whole protein) | GenBank AAH93061.1 |
| | | | or |
| | | | UniProtKB/Swiss -Prot: Q13630 (GDP L-fucose synthase) |
| 14 | AAAAARPAGGSARRWGRPGRCGLLAAGPKRVR SEPGGRLPERSLGPAHPAPAAMAGTLDLDKGCT VEELLRGCIEAFDDSGKVRDPQLVRMFLMMHPW YIPSSQLAAKLLHIYQQSRKDNSNSLQVKTCHLVR YWISAFPAEFDLNPELAEQIKELKALLDQEGNRR HSSLIDIDSVPTYKWKRQVTQRNPVGQKKRKMSL LFDHLEPMELAEHLTYLEYRSFCKILFQDYHSFVT HGCTVDNPVLERFISLFNSVSQWVQLMILSKPTA PQRALVITHFVHVAEKLLQLQNFNTLMAVVGGLS HSSISRLKETHSHVSPETIKLWEGLTELVTATGNY GNYRRRLAACVGFRFPILGVHLKDLVALQLALPD | 1-662 (whole protein) | GenBank AAI10307.1 (RASGRP2) |
| | WLDPARTRLNGAKMKQLFSILEELAMVTSLRPPV QANPDLLSLLTVSLDQYQTEDELYQLSLQREPRS KSSPTSPTSCTPPPRPPVLEEWTSAAKPKLDQAL VVEHIEKMVESVFRNFDVDGDGHISQEEFQIIRGN FPYLSAFGDLDQNQDGCISREEMVSYFLRSSSVL GGRMGFVHNFQESNSLRPVACRHCKALILGIYKQ GLKCRACGVNCHKQCKDRLSVECRRRAQSVSLE GSAPSPSPMHSHHHRAFSFSLPRPGRRGSRPPE IREEEVQTVEDGVFDIHL | | |
| 15 | | 1-609 (whole protein) | UniProtKB/Swiss -Prot Q7LDG7-1 |
| | | | or |
| | | | GenPept NP_722541.1 (RASGRRP2 Isoform 1) |
| 16 | MGTQRLCGRGTQGWPGSSEQHVQEATSSAGLH SGVDELGVRSEPGGRLPERSLGPAHPAPAAMAG TLDLDKGCTVEELLRGCIEAFDDSGKVRDPQLVR MFLMMHPWYIPSSQLAAKLLHIYQQSRKDNSNSL QVKTCHLVRYWISAFPAEFDLNPELAEQIKELKAL LDQEGNRRHSSLIDIDSVPTYKWKRQVTQRNPV GQKKRKMSLLFDHLEPMELAEHLTYLEYRSFCKI | 1-671 (whole protein) | UniProtKB/Swiss -Prot Q7LDG7-2 |
| | | | or |
| | | | GenPept AAF07219.1 (RASGRRP2 Isoform 2) |
| | LFQDYHSFVTHGCTVDNPVLERFISLFNSVSQWV QLMILSKPTAPQRALVITHFVHVAEKLLQLQNFNT LMAVVGGLSHSSISRLKETHSHVSPETIKLWEGLT ELVTATGNYGNYRRRLAACVGFRFPILGVHLKDL VALQLALPDWLDPARTRLNGAKMKQLFSILEELA MVTSLRPPVQANPDLLSLLTVSLDQYQTEDELYQ LSLQREPRSKSSPTSPTSCTPPPRPPVLEEWTSA AKPKLDQALVVEHIEKMVESVFRNFDVDGDGHIS QEEFQIIRGNFPYLSAFGDLDQNQDGCISREEMV SYFLRSSSVLGGRMGFVHNFQESNSLRPVACRH CKALILGIYKQGLKCRACGVNCHKQCKDRLSVEC RRRAQSVSLEGSAPSPSPMHSHHHRAFSFSLPR PGRRGSRPPEIREEEVQTVEDGVFDIHL | | |
| 17 | MAGTLDLDKGCTVEELLRGCIEAFDDSGKVRDP QLVRMFLMMHPWYIPSSQLAAKLLHIYQQSRKDN SNSLQVKTCHLVRYWISAFPAEFDLNPELAEQIKE LKALLDQEGNRRHSSLIDIDSVCVGAEHRGLGGH SVSYTICA | 1-144 (whole protein) | UniProtKB/Swiss -Prot Q7LDG7-3 (RASGRRP2 Isoform 3) |
| 18 | MAGTLDLDKGCTVEELLRGCIEAFDDSGKVRDP QLVRMFLMMHPWYIPSSQLAAKLLHIYQQSRKDN SNSLQVKTCHLVRYWISAFPAEFDLNPELAEQIKE LKALLDQEGNRRHSSLIDIDSVPTYKWKRQVTQR NPVGQKKRKMSLLFDHLEPMELAEHLTYLEYRSF CKILFQDYHSFVTHGCTVDNPVLERFISLFNSVSQ WVQLMILSKPTAPQRALVITHFVHVAEKLLQLQNF NTLMAVVGGLSHSSISRLKETHSHVSPETIKLWE GLTELVTATGNYGNYRRRLAACVGFRFPILGVHL KDLVALQLALPDWLDPARTRLNGAKMKQLFSILE ELAMVTSLRPPVQANPDLLSLLTVSLDQYQTEDE LYQLSLQREPRSKSSPTSPTSCTPPPRPPVLEEW TSAAKPKLDQALVVEHIEKMVESVFRNFDVDGDG HISQEEFQIIRGNFPYLSAFGDLDQNQDGCISREE MVSYFLRSSSVLGGRMGFVHNFQESNSLRPVAC RHCKALILGIYKQGLKCRACGVNCHKQCKDRLSV | 1-610 (whole protein) | UniProtKB/Swiss -Prot Q7LDG7-4 |
| | | | or |
| | | | GenPept XP _ 011543022.1 (RASGRRP2 Isoform 4) |
| | ECRRRAQSVSLEGSAPSPSPMHSHHHRAFSFSL PRPGRRGSRPPAEIREEEVQTVEDGVFDIHL | | |
| 19 | MASQKRPSQRHGSKYLATASTMDHARHGFLPR HRDTGILDSIGRFFGGDRGAPKRGSGKDSHHPA RTAHYGSLPQKSHGRTQDENPVVHFFKNIVTPRT PPPSQGKGRGLSLSRFSWGAEGQRPGFGYGGR ASDYKSAHKGFKGVDAQGTLSKIFKLGGRDSRS GSPMARR | 1-171 (whole protein) | UniProtKB/Swiss -Prot P02686-5 (MBP Isoform 5) |
| 20 | MASLSRPSLPSCLCSFLLLLLLQVSSSYAGQFRVI GPRHPIRALVGDEVELPCRISPGKNATGMEVGW YRPPFSRVVHLYRNGKDQDGDQAPEYRGRTELL KDAIGEGKVTLRIRNVRFSDEGGFTCFFRDHSYQ EEAAMELKVEDPFYWVSPGVLVLLAVLPVLLLQIT VGLIFLCLQYRLRGKLRAEIENLHRTFDPHFLRVP CWKITLFVIVPVLGPLVALIICYNWLHRRLAGQFLE ELRNPF | 1-247 (whole protein) | UniProtKB/Swiss -Prot Q16653-1 (MOG Isoform 1) |
| 21 | | 1-277 (whole protein) | UniProtKB/Swiss -Prot P60201-1 (PLP Isoform 1) |
| 22 | LVRYWISAFP | 131-140 | GenBank AAI10307.1 (RASGRP2) |
| | | or 78-87 | or |
| | | | UniProtKB/Swiss -Prot Q7LDG7-1 (RASGRP2 Isoform 1) |
| 23 | SLQVKTCHLVRYWISAFPAEFDLNPE | 123-148 | GenBank AAI10307.1 (RASGRP2) |
| | | or 70-95 | or |
| | | | UniProtKB/Swiss -Prot Q7LDG7-1 (RASGRP2 Isoform 1) |
| 24 | LVRYWISAFPAEFDLNPE | 131-148 | GenBank AAI10307.1 (RASGRP2) |
| | | or 78-95 | or |
| | | | UniProtKB/Swiss -Prot Q7LDG7-1 (RASGRP2 Isoform 1) |
| 25 | SNSLQVKTCHLVRYWISAFPAEFDLNPELA | 121-150 | GenBank AAI10307.1 (RASGRP2) |
| | | or 68-97 | or |
| | | | UniProtKB/Swiss -Prot Q7LDG7-1 (RASGRP2 Isoform 1) |
| 26 | LVRYWISAFPAEFDL | 131-145 | GenBank AAI10307.1 (RASGRP2) |
| | | or 78-92 | or |
| | | | UniProtKB/Swiss -Prot Q7LDG7-1 (RASGRP2 Isoform 1) |
| 27 | EEDEVSIKEAAEAVV | 246-260 | GenBank AAH93061.1 |
| | | | (GDP L-fucose synthase) |
| 28 | EEDEVSIKEAAEAVVEAMDF | 246-265 | GenBank AAH93061.1 (GDP L-fucose synthase) |
| 29 | DEVSIKEAAEAVVEAMDFHGE | 248-268 | GenBank AAH93061.1 (GDP L-fucose synthase) |
| 30 | EDEVSIKEAAEAVVEAMDFHGEV | 247-269 | GenBank AAH93061.1 (GDP L-fucose synthase) |
| 31 | EEDEVSIKEAAEAVVEAMDFHGEVT | 246-270 | GenBank AAH93061.1 (GDP L-fucose synthase) |
| 32 | SIKEAAEAVVEAMDF | 251-265 | GenBank AAH93061.1 (GDP L-fucose synthase) |

Possible polypeptides or proteins are listed in Table 2:

**Table 2: Polypeptides or proteins comprising at least one peptide. The sequences are human sequences.**

| SEQ ID NO: | Sequence (amino acids) | Peptides comprised (SEQ ID NO) in the order listed |
|---|---|---|
| 33 | KYLATASTMDHARHGFLPRHGGSENPVVHFFKNIV TPRTPGGSLSRFSWGAEGQRPGFGYGGGGSAQ GTLSKIFKLGGRDSRSGSPMARRGGSGQFRVIGP RHPIRALVGDEVGGSMEVGWYRPPFSRVVHLYRN GKGGSHCLGKWLGHPDKFVGIGGSTAQTRALFEK | MBP1 (3), MBP2 (4), MBP3 (5), MBP4 (6), MOG1 (7), MOG2 (8), PLP1 (9), TSTA3-1 (10), TSTA3-2 (11), TSTA3-3 |
| | VQPTHGGSPHNSNFGYSYAKRMIGGSLSVGEEDE VSGGSFRFTPFKQAVKETCAGGSKTCHLVRYWIS AFPAEFD | (1), TSTA3-4 (12), RASGRP2 (2) with GGS linker between each peptide |
| 34 | KYLATASTMDHARHGFLPRHENPVVHFFKNIVTPR TPLSRFSWGAEGQRPGFGYGGAQGTLSKIFKLGG RDSRSGSPMARRGQFRVIGPRHPIRALVGDEVME VGWYRPPFSRVVHLYRNGKHCLGKWLGHPDKFV GITAQTRALFEKVQPTHPHNSNFGYSYAKRMILSV GEEDEVSFRFTPFKQAVKETCAKTCHLVRYWISAF PAEFD | MBP1 (3), MBP2 (4), MBP3 (5), MBP4 (6), MOG1 (7), MOG2 (8), PLP1 (9), TSTA3-1 (10), TSTA3-2 (11), TSTA3-3 (1), TSTA3-4 (12), RASGRP2 (2) without linker |
| 35 | KYLATASTMDHARHGFLPRHGGSENPVVHFFKNIV TPRTPGGSLSRFSWGAEGQRPGFGYGGGGSAQ GTLSKIFKLGGRDSRSGSPMARRGGSGQFRVIGP RHPIRALVGDEVGGSMEVGWYRPPFSRVVHLYRN GK | MBP1 (3), MBP2 (4), MBP3 (5), MBP4 (6), MOG1 (7), MOG2 (8) with GGS linker between each peptide |
| 36 | HCLGKWLGHPDKFVGIGGSTAQTRALFEKVQPTH GGSPHNSNFGYSYAKRMIGGSLSVGEEDEVSGGS FRFTPFKQAVKETCAGGSKTCHLVRYWISAFPAEF D | PLP1 (9), TSTA3-1 (10), TSTA3-2 (11), TSTA3-3 (1), TSTA3-4 (12), RASGRP2 (2) with GGS linker between each peptide |
| 37 | HCLGKWLGHPDKFVGIGGSENPVVHFFKNIVTPRT PGGSMEVGWYRPPFSRVVHLYRNGKGGSTAQTR ALFEKVQPTHGGSKYLATASTMDHARHGFLPRHG GSPHNSNFGYSYAKRMIGGSLSRFSWGAEGQRP GFGYGGSLSVGEEDEVSGGSAQGTLSKIFKLGGR DSRSGSPMARRGGSKTCHLVRYWISAFPAEFDGG SGQFRVIGPRHPIRALVGDEVGGSFRFTPFKQAVK ETCA | PLP1 (9), MBP2 (4), MOG2 (8), TSTA3-1 (10), MBP1 (3), TSTA3-2 (11), MBP3 (5), TSTA3-3 (1), MBP4 (6), RASGRP2 (2), MOG1 (7), TSTA3-4 (12) with S or GGS linker between each peptide |

In a preferred embodiment, the polypeptide or protein has one of the sequences as defined by SEQ ID NOs: 33 to 37, preferably SEQ ID NO: 37. A His Tag, for example a 6 His Tag (consisting of six histidine residues) can be added, for example at the N-terminus, for easier isolation. Labelling is for example possible with biotin, for example on at least one cysteine.

A nucleic acid encoding the peptide and/or the combination and/or the polypeptide or protein as disclosed *supra* is also provided. The nucleic acid encoding the peptide and/or the combination and/or the polypeptide or protein preferably refers to any coding nucleotide sequence, for example RNA or DNA, in particular mRNA or cDNA. In one embodiment, the nucleotide sequence is a plasmid or any type of vector known to the person skilled in the art. In a preferred embodiment, the nucleotide sequences do not comprise introns and the gene sequences comprise exons and introns.

The following gene sequences (Table 3) represent the human gene sequences of the proteins GDP-L fucose synthase (gene: TSTA3), RASGRP2, MBP, MOG and PLP (Table 3). The peptide and/or the combination and/or the polypeptide or protein as disclosed *supra* is preferably encoded by the respective gene.

**Table 3: Gene sequences and database entry. The sequences are human sequences.**

| Gene name | Database entry |
|---|---|
| TSTA3 | NCBI Reference Sequence: NC_000008.11 |
| | REGION: 143612618..143618048 |
| RASGRP2 | NCBI Reference Sequence: NC_000011.10 |
| | REGION: 64726911..64745456 |
| MBP | NCBI Reference Sequence: NC_000018.10 |
| | REGION: 76978833..77133708 |
| MOG | NCBI Reference Sequence: NC_000006.12 |
| | REGION: 29657092..29672365 |
| PLP | NCBI Reference Sequence: NC_000023.11 |
| | REGION: 103776506..103792619 |

The following nucleotide sequences (Table 4) represent preferred nucleotide sequences encoding any of the peptides and/or combinations and/or polypeptides or proteins as disclosed *supra* and those encoding the corresponding peptides and/or combinations and/or polypeptides or proteins in *Mus musculus* and *Rattus norvegicus.* The table also comprises coding sequences (CDS), i.e. proteins or peptides of *Mus musculus* and *Rattus norvegicus,* which can also be used in the context of the present invention, i.e. which comprise the *Mus musculus* and *Rattus norvegicus* peptides and/or combinations and/or polypeptides or proteins corresponding to the human peptides and/or combinations and/or polypeptides or proteins of the present invention. Corresponding nucleotide sequences, peptides, combinations and/or polypeptides or proteins in other Rodentia which are not included in the table are also encompassed by the present invention and can be retrieved from the person skilled in the art from the respective databases.

**Table 4: Nucleotide sequences and protein sequences and the respective database entries (NCBI Accession Nos for RNA and UniProt Accession Nos for protein if not indicated otherwise). The species is indicated.**

| Gene name | Database entry | Species |
|---|---|---|
| TSTA3 | RNA: | |
| | XM_011517269.1 | Homo sapiens |
| | NM_003313.3 | Homo sapiens |
| | NM_001317783.1 | Homo sapiens |
| | XM_005251051.3 | Homo sapiens |
| | NM_031201.2 | Mus musculus |
| | NM_001357005.2 | Mus musculus |
| | NM_001411815.1 | Mus musculus |
| | NM_001411816.1 | Mus musculus |
| | ENSMUSG00000022570.8 (Ensembl) | Mus musculus |
| | NM_001270789.1 | Rattus norvegicus |
| | NM_001127455.2 | Rattus norvegicus |
| | NM_001270790.1 | Rattus norvegicus |
| | BC158885.1 (GenBank) protein: | Rattus norvegicus |
| | P23591 | Mus musculus |
| | A0A2R8VI39 | Mus musculus |
| | A0A2R8VHD0 | Mus musculus |
| | A0A2R8VKL9 | Mus musculus |
| | A0A2R8W6P6 | Mus musculus |
| | A0A2R8W6N0 | Mus musculus |
| | B0BNN0 | Rattus norvegicus |
| | G3V762 | Rattus norvegicus |
| RASGRP2 | RNA: | |
| | XM_011544722.2 | Homo sapiens |
| | NM_153819 | Homo sapiens |
| | XM_011544723.3 | Homo sapiens |
| | XM_011544721.1 | Homo sapiens |
| | NM_001098670.1 | Homo sapiens |
| | XM_017017084.2 | Homo sapiens |
| | XM_011544720.2 | Homo sapiens |
| | XM_011544718.2 | Homo sapiens |
| | XM_017017082.2 | Homo sapiens |
| | XM_005273707.4 | Homo sapiens |
| | XM_017017083.2 | Homo sapiens |
| | NM_001318398.1 | Homo sapiens |
| | NM_001098671.1 | Homo sapiens |
| | XM_011544725.2 | Homo sapiens |
| | XM_017017085.2 | Homo sapiens |
| | XM_017017086.1 | Homo sapiens |
| | XR_001747720.2 | Homo sapiens |
| | XR_001747719.2 | Homo sapiens |
| | NM_011242.4 | Mus musculus |
| | XM_006531686.5 | Mus musculus |
| | XM_006531688.3 | Mus musculus |
| | XM_006531687.2 | Mus musculus |
| | NM_001411231.1 | Mus musculus |
| | NM_001411232.1 | Mus musculus |
| | XM_006531690.3 | Mus musculus |
| | XM_006531691.3 | Mus musculus |
| | ENSMUSG00000032946.17 (Ensembl) | Mus musculus |
| | ENSRNOG00000021098.7 (Ensembl) | Rattus norvegicus |
| | NM_001082977.2 | Rattus norvegicus |
| | XM_006230854.4 | Rattus norvegicus |
| | XM_006230857.3 | Rattus norvegicus |
| | XM_006230855.4 protein: | Rattus norvegicus |
| | Q9QUG9 | Mus musculus |
| | D3YZP1 | Mus musculus |
| | D3YZP4 | Mus musculus |
| | D3Z2F9 | Mus musculus |
| | D3Z3N3 | Mus musculus |
| | D6RDD3 | Mus musculus |
| | D3YZX2 | Mus musculus |
| | D3YXD6 | Mus musculus |
| | D3YZP2 | Mus musculus |
| | P0C643 | Rattus norvegicus |
| | R9PXW6 | Rattus norvegicus |
| | A0A8I5ZQH7 | Rattus norvegicus |
| MBP | RNA: | |
| | NM_001025081.2 | Homo sapiens |
| | NM_001025090.2 | Homo sapiens |
| | NM_001025092.2 | Homo sapiens |
| | NM_001025100.2 | Homo sapiens |
| | NM_001025101.2 | Homo sapiens |
| | NM_002385.3 | Homo sapiens |
| | NM_001025251.2 | Mus musculus |
| | NM_001025254.2 | Mus musculus |
| | NM_001025255.2 | Mus musculus |
| | NM_001025256.2 | Mus musculus |
| | NM_001025258.2 | Mus musculus |
| | NM_001025259.2 | Mus musculus |
| | NM_010777.3 | Mus musculus |
| | NM_001025245.1 | Mus musculus |
| | MH926013.1 (GenBank) | Mus musculus |
| | DQ887821.1 (GenBank) | Mus musculus |
| | ENSMUSG00000041607.18 (Ensembl) | Mus musculus |
| | NM_001025291.1 | Rattus norvegicus |
| | NM_001025292.1 | Rattus norvegicus |
| | NM_001025293.1 | Rattus norvegicus |
| | NM_001025294.1 | Rattus norvegicus |
| | NM_017026.2 | Rattus norvegicus |
| | AJ132895.1 (GenBank) | Rattus norvegicus |
| | AJ132896.1 (GenBank) | Rattus norvegicus |
| | AJ132897.1 (GenBank) | Rattus norvegicus |
| | AJ132898.1 (GenBank) | Rattus norvegicus |
| | AF439750.1 (GenBank) | Rattus norvegicus |
| | M25889.1 (GenBank) | Rattus norvegicus |
| | BC094522.1 (GenBank) protein: | Rattus norvegicus |
| | P04370 | Mus musculus |
| | A0A411P8U6 | Mus musculus |
| | Q09J72 | Mus musculus |
| | A0A498WGS3 | Mus musculus |
| | Q3UGF3 | Mus musculus |
| | Q5D096 | Mus musculus |
| | P02688 | Rattus norvegicus |
| MOG | RNA: | |
| | NM_001008228.3 | Homo sapiens |
| | NM_001008229.3 | Homo sapiens |
| | NM_001170418.2 | Homo sapiens |
| | NM_001363610.2 | Homo sapiens |
| | NM_002433.5 | Homo sapiens |
| | NM_206809.4 | Homo sapiens |
| | NM_206810.4 | Homo sapiens |
| | NM_206811.4 | Homo sapiens |
| | NM_206812.4 | Homo sapiens |
| | NM_206814.6 | Homo sapiens |
| | NM_010814.2 | Mus musculus |
| | AY566830.1 | Mus musculus |
| | L21995.1 | Rattus norvegicus |
| | M99485.1 | Rattus norvegicus |
| | NM_022668.2 | Rattus norvegicus |
| | ENSRNOG00000000775.4 (Ensembl) protein: | Rattus norvegicus |
| | Q61885 | Mus musculus |
| | Q3UY21 | Mus musculus |
| | Q29ZQ5 | Mus musculus |
| | Q63345 | Rattus norvegicus |
| | Q6M FX9 | Rattus norvegicus |
| | A0A8I6A3S4 | Rattus norvegicus |
| | A0A8I5ZLK9 | Rattus norvegicus |
| PLP | RNA: | |
| | NM_000533.5 | Homo sapiens |
| | NM_001128834.3 | Homo sapiens |
| | NM_001305004.1 | Homo sapiens |
| | NM_199478.3 | Homo sapiens |
| | NM_001290561.2 | Mus musculus |
| | NM_011123.4 | Mus musculus |
| | NM_001359117.1 | Mus musculus |
| | NM_001359120.1 | Mus musculus |
| | NM_001359118.1 | Mus musculus |
| | XM_017601914.2 | Rattus norvegicus |
| | KJ841934.1 (GenBank) | Rattus norvegicus |
| | CH474076.1 (GenBank) | Rattus norvegicus |
| | NM_030990.3 | Rattus norvegicus |
| | XM_017601913.2 | Rattus norvegicus |
| | X02809.1 | Rattus norvegicus |
| | M11185.1 | Rattus norvegicus |
| | BC093596.1 protein: | Rattus norvegicus |
| | P60202 | Mus musculus |
| | Q3UYM8 | Mus musculus |
| | A0A097BVK2 | Rattus norvegicus |
| | P60203 | Rattus norvegicus |

All *Mus musculus* and *Rattus norvegicus* sequences were retrieved from the respective online databases on June 26, 2023, the sequences of *Homo sapiens* were retrieved on June 22, 2018 (TSTA3 and RASGRP2) and on September 19, 2023 (MBP, MOG, PLP).

In one embodiment, the invention relates to a carrier comprising the peptide and/or the combination and/or the polypeptide or protein and/or the nucleic acid according to the present invention.

A carrier in the sense of the present invention is preferably adapted to or is capable of carrying or transporting the peptide and/or the combination and/or the polypeptide or protein and/or the nucleic acid, preferably *in vivo.* In order to carry or transport the peptide and/or the combination and/or the polypeptide or protein and/or the nucleic acid, the carrier is preferably directly or indirectly coupled to the peptide and/or the combination and/or the polypeptide or protein and/or contains the nucleic acid.

The carrier is preferably selected from the group consisting of a cell or a population of cells, a membrane of a cell, a protein, a lipid, a glycolipid, a bead, a nanoparticle, a virus-like-particle (VLP) and a molecule, such as a sugar molecule, and any combination thereof. Preferably, the carrier is a cell, more preferably a blood cell, more preferably a red or white blood cell, even more preferably an RBC, or a population of cells, more preferably a population of blood cells, more preferably a population of red or white blood cells, even more preferably a population of RBCs. In case a membrane of a cell is selected, a membrane of an RBC is preferred. The term cell also includes cell precursors, e.g. RBC precursors.

In a very preferred embodiment, red blood cells (RBCs), in particular autologous RBCs are used as carriers. These cells are advantageous because 1) there is extensive experience with the use of RBCs in medicine, which is an important safety aspect; 2) procedures for collection and storage of RBCs are well established in clinical routine; 3) RBCs target the liver as a tolerogenic organ in addition to the spleen as a tolerogenic organ; 4) several products can be manufactured from a single blood donation.

In one embodiment, nucleated cells, e.g. nucleated RBCs, are used, which are intact cells comprising a nucleus. In another embodiment, enucleated cells, e.g. enucleated RBCs, are used. Enucleated cells are for example obtained by enucleation during differentiation and maturation from progenitor cells. Preferably, nucleated cells, more preferably nucleated RBCs are used.

Allogeneic cells, preferably allogeneic red blood cells may also be used. Autologous refers to cells that are obtained from the same subject who will receive the cells, while allogeneic refers to cells that are obtained from a different subject, preferably of the same species. Preferably, allogeneic cells are matched with patients sharing the same or a compatible blood group to avoid incompatibility. The person skilled in the art knows which blood groups are compatible.

It is particularly preferred that the carrier is coupled to the peptide and/or the combination and/or the polypeptide or protein and/or that the carrier contains the nucleic acid according to the present invention.

In one preferred aspect of the invention, a cell or a population of cells is provided (i.e. the carrier is a cell or population of cells) which is chemically coupled to the peptide and/or the combination and/or the polypeptide or protein.

The cell or population of cells can be coupled to the peptide and/or the combination and/or the polypeptide or protein by a coupling process, e.g. by a chemical coupling process, preferably by 1-ethyl-3-(3-dimethylaminopropyl)-carbodiimide (ECDI/EDC). The terms ECDI and EDC are used interchangeably herein. Preferably, the cell is a blood cell, even more preferably a red or white blood cell or the population of cells is a population of blood cells, even more preferably a population of red or white blood cells. The white blood cell (by itself or within a population) may be a splenocyte or a PBMC or generally an APC. Most preferred is an RBC or a population of RBCs. Hence, a cell, in particular a red or white blood cell, preferably an RBC, or a population of cells, in particular a population of red or white blood cells, preferably a population of RBCs chemically coupled to the peptide and/or the combination and/or the polypeptide or protein by EDC is preferred.

The population of cells may be heterogenous in that the cells within a population may differ with respect to the number and kind of coupled peptides and/or polypeptides or proteins.

In one embodiment, a cell or a population of cells is provided, wherein the cell or population of cells is an RBC or a population of RBCs, chemically coupled to at least the peptide TSTA3-3 (242-251) (SEQ ID NO: 1).

In another embodiment, a cell or a population of cells is provided, wherein the cell or population of cells is an RBC or a population of RBCs, chemically coupled to at least the peptide RASGRP2 (74-91) (SEQ ID NO: 2).

In a very preferred embodiment, an RBC or a population of RBCs is provided which is chemically coupled to the combination comprising or consisting of the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12). Preferably, each peptide is coupled at least once.

Preferably, at least one peptide coupled to the carrier is biotinylated. For example, RASGRP2 (74-91) (SEQ ID NO: 2) and/or TSTA3-3 (242-251) (SEQ ID NO: 1) is biotinylated, preferably RASGRP2 (74-91) (SEQ ID NO: 2) is biotinylated.

In one embodiment, the carrier is an RBC or a population of RBCs, chemically coupled to at least one polypeptide or protein comprising or consisting of a sequence as set forth in any one of SEQ ID NOs: 33-37, for example comprising or consisting of a sequence as set forth in SEQ ID NO: 37. In one embodiment, at least one polypeptide or protein is biotinylated, preferably the polypeptide or protein comprising or consisting of a sequence as set forth in SEQ ID NO: 37 is biotinylated.

In one embodiment, the peptide, combination, polypeptide or protein is expressed by the cell, preferably the blood cell. Thereby, the genetic information encoding the peptide/s and/or polypeptide or protein is introduced into the cell before the peptide/s and/or combination and/or polypeptide or protein is expressed by the cell.

A method of manufacturing the carrier comprising providing the carrier, adding the peptide and/or the combination and/or the polypeptide or protein as disclosed *supra* and a coupling reagent is also provided. The carrier may be any carrier as disclosed *supra.* Preferably, the carrier is a cell, more preferably a blood cell, even more preferably an RBC. In particular, a method of manufacturing a chemically coupled cell (i.e. antigen-coupled cell) or population of cells is provided (i.e. the carrier is a cell or a population of cells) comprising providing an isolated cell, preferably a blood cell, preferably an RBC, or a population of isolated cells, preferably a population of blood cells, preferably a population of RBCs, from a human subject, adding the peptide and/or the combination and/or the polypeptide or protein (i.e., the antigen) as disclosed *supra* and subsequently adding a coupling agent.

Any coupling agent or method for coupling a peptide and/or combination of peptides and/or polypeptide or protein to a carrier, preferably a cell or population of cells, may be used. For example, a synthetic or natural linker may be employed for coupling. One example of such a linker is glycophorin A, present on the surface of RBCs. In one embodiment, chemical crosslinking is performed. In a preferred embodiment, the chemical crosslinker EDC is used. EDC catalyzes the formation of peptide bonds between free amino and carboxyl groups. EDC can be used for coupling to any carrier, preferably a cell or population of cells, as long as a free amino group and/or free carboxy group is present. Particularly in the presence of EDC, multiple peptides and/or combination of peptides and/or polypeptides or proteins can be coupled to the surface of the cell thereby allowing for the simultaneous targeting of multiple T cell specificities.

Preferably, more than three, more than five, more than 10, more than 15, or even more than 20 different peptides and/or polypeptides or proteins are coupled to the surface of the carrier, preferably the cell or population of cells. In one embodiment, a maximum amount of 25 different peptides is added. In one embodiment, a maximum amount of five different polypeptides or proteins is added. In a preferred embodiment, between five and 20, preferably between five and 15 different peptides and/or polypeptide or proteins are added. In a particularly preferred embodiment, 12 different peptides are added. A peptide or polypeptide or protein is different from another peptide or polypeptide or protein if it does not consist of the same amino acid sequence. It is also possible that the same peptide and/or polypeptide or protein is coupled more than once to the same carrier, preferably the cell or population of cells.

In another embodiment, a combination of peptides is coupled to the carrier, preferably the cell or population of cells. It is particularly advantageous to combine at least one peptide of the protein TSTA-3 and/or RASGRP2 according to the present invention, particularly a peptide as defined by one of the sequences with SEQ ID NO: 1, 2, 10, 11 or 12, with at least one myelin peptide, in particular with at least one or all of the myelin peptides as defined by SEQ ID NOs: 3 to 9.

In a preferred embodiment, a method of manufacturing a chemically coupled carrier, preferably a chemically coupled cell or population of cells, is provided, wherein a combination comprising the peptide TSTA3-3 (242-251) (SEQ ID NO: 1) and/or the peptide RASGRP2 (74-91) (SEQ ID NO: 2), and at least one peptide selecting from the group consisting of MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12) is coupled.

In another preferred embodiment, a method of manufacturing a chemically coupled carrier, preferably a chemically coupled cell or population of cells, is provided, wherein a combination of peptides comprising or consisting of TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12) is coupled. Preferably, the combination of peptides consists of TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12) and RASGRP2 (74-91) (SEQ ID NO: 2) is biotinylated.

The mechanism of action of cells coupled with peptide/polypeptide/protein by EDC is not fully understood, but involves covalently linking amino- and carboxy groups of peptide/polypeptide/protein and cell surface molecules, subsequent programmed cell death (apoptosis in the case of nucleated cells; eryptosis in the case of RBC) of the peptide/polypeptide/protein-coupled, i.e. antigen-coupled, cells and then tolerogenic presentation of the dying cells *in vivo.*

The cell number used in the coupling reaction may greatly vary and may be adjusted according to the EDC and peptide (or polypeptide or protein) concentration. Preferably, 3×10⁷ to 5×10¹⁰ RBCs/ml are provided, more preferably 3×10⁸ to 3×10¹⁰ RBCs/ml, more preferably about 3×10⁹ RBCs/ml. For example, about 3×10⁷ or 3×10⁸ or 2×10⁹ or 3×10⁹ or 3×10¹⁰ RBCs/ml may be used in a coupling reaction, for example 3×10⁹ RBCs/ml with 3mg/ml EDC-HCl and 7 different peptides or 3×10¹¹ RBCs with 3mg/ml EDC-HCI and 12 different peptides. In a preferred embodiment, an amount of 3×10¹¹ RBCs is used for the coupling reaction. The cell number may also be higher or lower depending on the specific need.

The peptide and/or combination of peptides and/or polypeptide or protein may be added in an amount to be readily determined by the person skilled in the art. The person skilled is aware of measures to determine the optimal amount in the interplay with an optimal amount of EDC. The person skilled in the art can choose a suitable concentration based on, for example, coupling efficiency and viability of the cells. The final peptide concentration (i.e. the concentration in the coupling assay) used for coupling may greatly vary, for example between 0.002 mg/ml per peptide and 2 mg/ml per peptide, in particular between 0.1 mg/ml per peptide and 1 mg/ml per peptide, for example at a concentration of about 0.002 mg/ml, 0.007 mg/ml, 0.05 mg/ml, 0.07 mg/ml, 0.125 mg/ml, 0.132 mg/ml, 0.250 mg/ml, 0.5 mg/ml, 0.7 mg/ml, 1 mg/ml, 1.5 mg/ml or 2 mg/ml per peptide. A concentration of 0.132 mg/ml per peptide is especially preferred, particularly when 12 peptides are coupled.

The concentration of polypeptide or protein may also vary. Preferably, between 0.01 mg/ml and 10 mg/ml of polypeptide or protein, more preferably between 0.5 mg/ml and 5 mg/ml, are added in the coupling reaction. Preferably, a maximum amount of 5 different polypeptides or proteins is added.

The suitable concentrations may also depend on the total amount of different peptides or polypeptides or proteins coupled, i.e. the concentration of all peptides and/or polypeptides and/or proteins together. Preferably, a total amount of less than 100 mg/ml, more preferably less than 50 mg/ml, even more preferably less than 20 mg/ml, even more preferably less than 15 mg/ml, in particular a maximum amount of 12 mg/ml of peptides and/or polypeptides and/or proteins is added.

The person skilled in the art can determine a suitable concentration. The cost of the peptide or polypeptide or protein is another factor taken into account when choosing the peptide concentration.

Peptides (peptide salts) and/or polypeptides or proteins can be used with counterions, for example acetate or trifluoroacetate (TFA), preferably acetate. Counter ions may have an influence on peptide solubility.

The dose of EDC used for the coupling reaction may be titrated to obtain a maximum of safety with best efficacy. In high concentrations, EDC may lead to lysis of cells, in particular of RBC. For optimal stability of the RBC, a final concentration of EDC of less than or equal to 30 mg/ml EDC is added, preferably less than or equal to 25 mg/ml, more preferably 0.5 to 20 mg/ml EDC, in particular 0.5 mg/ml, 1 mg/ml, 2 mg/ml, 3 mg/ml, 5 mg/ml, 10 mg/ml or 20 mg/ml EDC, more preferably 2 to 15 mg/ml, more preferably 3 to 10 mg/ml, even more preferably about 3 mg/ml EDC. In a particularly preferred embodiment, 3 mg/ml EDC are added.

The optimal dose may vary. The person skilled in the art knows how to determine the optimal stability of the RBC and the optimal dose of EDC for coupling. EDC is usually available as EDC-HCI.

In a preferred embodiment, an amount of 3×10¹¹ RBCs, 3 mg/ml EDC and 0.132 mg/ml for each of 12 peptides is used for the coupling reaction. The peptides are preferably TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12), wherein RASGRP2 (74-91) (SEQ ID NO: 2) is biotinylated.

In the coupling reaction, the pH may also be adjusted. Preferably, the coupling reaction is performed at a pH of pH 5-8.

The incubation time can be varied and tailored for each specific coupling reaction. In one embodiment, the coupling reaction is performed for about 15 min, about 30 min, about 45 min, about 60 min or about 120 min. Coupling efficiency may be better with longer time of incubation. In one embodiment, a maximum is reached after 60 min. Hence, in a particularly preferred embodiment, the coupling reaction is performed for about 60 min.

The incubation temperature may also vary. For example, 15-25°C or 2-8°C may be used. Coupling efficiency may be higher when the coupling reaction is performed at 15-25°C. Hence, in one embodiment, the coupling reaction is performed at 15 to 25°C.

Any excipient that allows the coupling reaction may be used. In one embodiment, the excipient is sterile and endotoxin free. In a preferred embodiment, the excipient is sterile, endotoxin free saline (NaCl 0.9%). Saline is approved for use in humans and provides a maximum of safety.

The person skilled in the art knows how to determine an optimal incubation time and temperature and how to determine possible excipients.

The coupling process may also be done with a carrier which is not a cell, for example protein, lipid, glycolipid, bead, nanoparticle, virus-like-particle (VLP), or molecule, such as a sugar molecule, or any combination thereof that is preferably suitable for application in humans and to which peptide/s and/or polypeptide/s and/or protein/s can be coupled by a coupling process, e.g. by a chemical coupling process, preferably by EDC. The carrier can be derived from one existing in nature or be synthetic.

Preferably, the cell, molecule, bead, nanoparticle, or VLP is biodegradable *in vivo* or is at least applicable to living subjects and broken down *in vivo* or is eliminated from the subject to which the carrier is applied.

In another aspect of the invention, a solution is provided comprising the peptide and/or the combination and/or the polypeptide or protein and/or the nucleic acid and/or the carrier (preferably the cell or population of cells) as disclosed *supra.* Preferably, the solution is isotonic. The solution is preferably suitable for infusions to a subject, in particular a human subject. In a preferred embodiment, the solution is saline or Ringer's lactate solution, preferably saline. Other solutions are also possible as long as the peptide and/or the combination and/or the polypeptide or protein and/or the nucleic acid and/or the carrier is soluble in the solution.

In one aspect of the invention, a pharmaceutical composition is provided which comprises the peptide and/or the combination and/or the polypeptide or protein and/or the nucleic acid and/or the carrier (preferably the cell or population of cells) and/or the solution according to the present invention, and a pharmaceutically acceptable excipient. A pharmaceutically acceptable excipient may be any chemical or adjuvant that can be administered to a human subject together with the peptide and/or combination of peptides and/or polypeptide or protein which for example serves the purpose of stabilizing the peptide and/or combination of peptides and/or polypeptide or protein. The pharmaceutically acceptable excipient may also support or enhance bioavailability, safety, effectiveness and/or delivery of the peptide and/or the combination and/or the polypeptide or protein and/or the nucleic acid and/or the carrier. The pharmaceutically acceptable excipient may also help maintain the integrity of the pharmaceutical composition during storage. Preferably, the pharmaceutically acceptable excipient does not alter or interfere with the structure and/or function of the peptide and/or the combination and/or the polypeptide or protein and/or the nucleic acid and/or the carrier.

In one aspect of the invention, the peptide and/or the combination and/or the polypeptide or protein and/or the nucleic acid and/or the carrier and/or the solution and/or the pharmaceutical composition as described *supra* is provided for use in the treatment and/or prevention of multiple sclerosis (MS). Treatment and/or prevention for example comprises administering the peptide and/or the combination and/or the polypeptide or protein and/or the nucleic acid and/or the carrier and/or the solution and/or the pharmaceutical composition to a subject, in particular to a human subject. Preferably, the carrier is a cell or a population of cells.

A method of treating, preventing and/or diagnosing MS is also provided comprising administering the peptide and/or the combination and/or the polypeptide or protein and/or the nucleic acid and/or the carrier and/or the solution and/or the pharmaceutical composition as disclosed *supra.* In another aspect, use of the peptide and/or the combination and/or the polypeptide or protein and/or the nucleic acid and/or the carrier and/or the solution and/or the pharmaceutical composition as disclosed *supra* is provided for the manufacture of a medicament for the treatment, prevention and/or diagnosis of MS. Treatment, prevention and/or diagnosis is preferably in a subject, more preferably in a human subject. Preferably, the carrier is a cell or a population of cells. Diagnosis may be performed *in vitro, in vivo* or ex *vivo* (see further *infra*)*.* In particular, pattern I and/or pattern II MS may be diagnosed.

Preferably, a therapeutically effective amount of the peptide and/or the combination and/or the polypeptide or protein and/or the nucleic acid and/or the carrier and/or the solution and/or the pharmaceutical composition is administered. Preferably, a cell or population of cells is administered which is coupled to a peptide, a combination of peptides and/or a polypeptide or protein.

One possible way of treatment and/or prevention is tolerance induction. Tolerance induction is antigen-specific and renders autoreactive T cells non-functional or anergic or alters their phenotype, specifically their cytokine secretion, or induces Treg cells that specifically suppress untoward autoimmunity to said target antigens. The induction of tolerance to target autoantigens is a highly important therapeutic goal in autoimmune diseases. It offers the opportunity to attenuate specifically the pathogenic autoimmune response in an effective way with few side effects. Tolerance induction can also be achieved by applying a whole protein instead of or in addition to an immunodominant peptide being a fragment of the protein (Kennedy et al., 1990, J Immunol, 144(3):909-15). It has herein been shown that immunologic changes consistent with tolerance can be induced in human patients upon i.v. injection of immunodominant peptides coupled to red blood cells. Hence, immunodominant peptides can be used for tolerance induction.

The immunodominance of the peptides and/or combination of peptides and/or polypeptides or proteins of the present invention thus allows using the peptides and/or combination of peptides and/or polypeptides or proteins for antigen-specific immunotherapies such as tolerance induction. Hence, the peptides of the present invention, either alone or in combination, or the polypeptides or proteins are useful for tolerance induction. They can for example be coupled to cells, for example RBCs.

Especially suitable for treatment and/or prevention, in particular tolerance induction, is a peptide comprising or consisting of the sequence of TSTA3-3 (242-251) (SEQ ID NO: 1) and/or a peptide comprising or consisting of the sequence of RASGRP2 (74-91) (SEQ ID NO: 2) and/or a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1 and/or SEQ ID NO: 2, respectively, wherein the peptide is soluble in a physiological solution and has a maximum length of 50 amino acids.

Also especially suitable is a peptide comprising or consisting of the sequence of RASGRP2 (74-91) (SEQ ID NO: 2) and/or a peptide comprising or consisting of a sequence with at least 80 %, preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 2, wherein the peptide has a maximum length of 50 amino acids.

The combination comprising one or more peptides as defined above and one or more of the following peptides:
a) a peptide comprising or consisting of the sequence of MBP1 (13-32) (SEQ ID NO: 3),
b) a peptide comprising or consisting of the sequence of MBP2 (83-99) (SEQ ID NO: 4),
c) a peptide comprising or consisting of the sequence of MBP3 (111-129) (SEQ ID NO: 5),
d) a peptide comprising or consisting of the sequence of MBP4 (146-170) (SEQ ID NO: 6),
e) a peptide comprising or consisting of the sequence of MOG1 (1-20) (SEQ ID NO: 7),
f) a peptide comprising or consisting of the sequence of MOG2 (35-55) (SEQ ID NO: 8),
g) a peptide comprising or consisting of the sequence of PLP1 (139-154) (SEQ ID NO:9),
h) a peptide comprising or consisting of the sequence of TSTA3-1 (51-65) (SEQ ID NO: 10),
i) a peptide comprising or consisting of the sequence of TSTA3-2 (136-150) (SEQ ID NO: 11),
j) a peptide comprising or consisting of the sequence of TSTA3-4 (296-310) (SEQ ID NO: 12), or
k) a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or SEQ ID NO: 12, respectively
is also particularly suitable for treatment and/or prevention of MS, in particular tolerance induction.

Particularly useful for treatment and/or prevention, in particular tolerance induction, in MS is a combination comprising or consisting of the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12). In a particularly preferred embodiment, RASGRP2 (74-91) (SEQ ID NO: 2) is biotinylated.

Antigen-specific tolerization preferably means a treatment or prevention strategy in autoimmune diseases which eventually leads to the subject less or not responding to self-antigens anymore (hence, being tolerant). Antigen-specific tolerization in MS with the peptides and/or combination of peptides and/or polypeptides or proteins of the present invention has multiple potential benefits for patients including: 1) It targets the specificity of autoreactive pathogenic T-cells, 2) multiple autoreactive T cell responses can be targeted at the same time, 3) it thus has the potential for long-term therapeutic benefit since autoreactive T-cells against existing epitopes may be tolerized. 4) Finally, a good safety profile can be presumed from prior data with a similar product (cf. EP 3 813 865).

An efficient antigen-specific tolerization, i.e. a lower inflammatory disease activity, can for example be measured by one or more of the following parameters, preferably measured during treatment phase vs. pre-treatment or baseline phase:
- a lower T2 lesion number and load on cerebral MRI scans
- a lower number of contrast enhancing lesions (CELs) on cerebral MRI scans
- a lower annualized relapse rate
- a lower mean Scripps Neurologic Rating Scale (SNRS)
- a lower mean Expanded Disability Status Scale (EDSS)
- a lower mean Multiple Sclerosis Functional Composite (MSFC)
- a lower rate of global or regional atrophy (measured by MRI)
- a decrease in iron ring-enhancing lesions as signs of chronic inflammation in the brain in MS lesions
- a decrease in antigen-specific T cell responses (for example monitored in serum and/or CSF by a Fluorospot assay with detection of the cytokines IFN-γ and/or IL-17A)
- an increase in T regulatory 1 (Tr1) cells (CD3+ CD4+ CD45RA- CD49b+, LAG3+) in serum and/or CSF
- an induction of nTreg cells in serum and/or CSF
- an increase of IL-10 and/or TGF-beta in serum and/or CSF
- a decrease in neurofilament light chain in serum
- a decrease of inflammatory- or glial activation markers such as chitinase 3-like protein 1, TREM-2 and/or GFAP.

The person skilled in the art is aware of methods and modes to measure the different parameters.

Hence, a successful treatment or prevention in the sense of the present invention preferably means that one or more of the following parameters is fulfilled indicating an active tolerance mechanism.

A particular advantage of a tolerization approach as disclosed herein is that it also has an effect on epitopes not contained in the vaccine (e.g. peptide-coupled cells), i.e. it inhibits epitope spreading. Hence, for example a personalized tolerization approach with a drug comprising red blood cells coupled to at least 2 peptides would be possible with the additional effect that tolerization is also achieved against epitopes that might have become a target in the future but which are not contained in the drug.

It has been successfully shown herein that peptide-coupled RBCs efficiently target liver and spleen of mice and humans rendering the coupled cells suitable and advantageous for tolerization.

In a preferred embodiment, a polypeptide or protein is used in a tolerization approach. Particularly preferred polypeptides or proteins are those comprising or consisting of at least one of the sequences as set forth in SEQ ID NOs: 33-37 or those comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with any one of the sequences of SEQ ID NOs: 33 to 37. A protein comprising or consisting of the sequence as set forth in SEQ ID NO: 37 is especially preferred, preferably a protein consisting of the sequence as set forth in SEQ ID NO: 37.

According to the present invention, antigen-specific tolerization can be used in all forms of MS: At the time of first manifestation, when differential diagnoses have been excluded, the disease is referred to as CIS provided that the CSF and MRI findings are consistent with the diagnosis. MRI discloses lesions in locations typical for MS, i.e. juxtacortical, periventricular, in the brain stem or spinal cord. If certain criteria are fulfilled that can be summarized as dissemination in space (more than one lesion or clinical symptom/sign) and time (more than one event) then the diagnosis of RRMS can be made. A special scenario is the accidental discovery of MRI lesions compatible with MS without clinical symptoms. This is referred to as RIS and can be considered a pre-stage of CIS and RRMS. More than 80% of patients suffer from one of these, and the majority of patients develops later what is called SPMS. At this time, relapses/exacerbations become less frequent or stop altogether and neurological disability increases steadily either between relapses or without these.

A special form of MS is PPMS, which never shows relapses, but rather begins with steady worsening of neurological symptoms, e.g. of the ability to walk. PPMS affects approximately 10% of MS patients and males and females with equal frequency. Its onset is usually later than CIS or RRMS. With respect to causes and disease mechanisms PPMS is considered similar to the above RIS-CIS-RRMS-SPMS.

Preferably, the tolerization approach is applied at an early stage, i.e. RIS, CIS and early RRMS, since it is assumed that the immune processes at this stage are primarily mediated by autoreactive T lymphocytes, while tissue damage, so-called degenerative changes, become gradually more important when the disease advances. However, tolerization is meaningful as long as there is an autoreactive T cell response against the antigens used for tolerization, which could also be during SPMS and PPMS.

Application at an early stage is particularly advantageous to avoid epitope spreading (cf. *supra*)*.* Hence, a treatment before epitope spreading has occurred would be desirable.

Hence, in one aspect of the invention, the peptide and/or the combination and/or the polypeptide or protein and/or the nucleic acid and/or the carrier and/or the solution and/or the pharmaceutical composition according to the present invention is provided for use in a method for inducing antigen-specific tolerance to autoantigens in a human subject suffering from or at risk of developing MS. Preferably, the carrier is a cell or a population of cells. The method preferably comprises the step of applying to a patient in the need thereof, i.e. to the human subject, the peptide and/or the combination and/or the polypeptide or protein and/or the nucleic acid and/or the carrier and/or the solution and/or the pharmaceutical composition according to the present invention.

In another aspect of the invention, a method for inducing antigen-specific tolerance to autoantigens in a human subject suffering from or at risk of developing MS is provided comprising administering the peptide and/or the combination and/or the polypeptide or protein and/or the nucleic acid and/or the carrier and/or the solution and/or the pharmaceutical composition according to the present invention.

Use of the peptide and/or the combination and/or the polypeptide or protein and/or the nucleic acid and/or the carrier and/or the solution and/or the pharmaceutical composition according to the present invention for the manufacture of a medicament for inducing antigen-specific tolerance to autoantigens in a human subject suffering from or at risk of developing MS is also provided.

The tolerization approach can thereby be used to prevent MS. This approach may include identifying those individuals (e.g. in a family with a MS patient), who are at a high risk of developing MS. For example, it is possible to tolerize e.g. the children of a mother with MS or the identical twin of a patient with MS, in whom the risk of developing MS would be particularly high.

In a particularly preferred aspect of the invention, the peptide and/or the combination and/or the polypeptide or protein and/or the nucleic acid and/or the carrier and/or the solution and/or the pharmaceutical composition according to the present invention is provided for use in a method for inducing antigen-specific tolerance to autoantigens in a human subject with early MS (i.e. RIS, CIS and early RRMS) or even pre-clinical stages of the disease. Preferably, the carrier is a cell or a population of cells.

In another aspect of the invention, a method for inducing antigen-specific tolerance to autoantigens in a human subject with early MS is provided comprising administering the peptide and/or the combination and/or the polypeptide or protein and/or the nucleic acid and/or the carrier and/or the solution and/or the pharmaceutical composition according to the present invention.

Use of the peptide and/or the combination and/or the polypeptide or protein and/or the nucleic acid and/or the carrier and/or the solution and/or the pharmaceutical composition according to the present invention for the manufacture of a medicament for inducing antigen-specific tolerance to autoantigens in a human subject with early MS is also provided.

The antigen-specific tolerance protocol provided herein may selectively target both activated and naive autoreactive T cells specific for multiple potential encephalitogenic epitopes that perpetuate the disease.

For the treatment and/or prevention, in particular for inducing antigen-specific tolerance to autoantigens, the following specificities for administration are preferred:
The peptide and/or the combination and/or the polypeptide or protein and/or the nucleic acid and/or the carrier and/or the solution and/or the pharmaceutical composition may be administered by subcutaneous (s.c.), intracoelomic (i.c), intramuscular (i.m.), intradermal (i.d.), transdermal (t.d.) or intravenous (i.v.) administration. Preferably, the carrier is a cell or a population of cells. The peptide and/or the combination and/or the polypeptide or protein and/or the solution and/or the pharmaceutical composition may also be administered by nasal, inhaled or oral administration. In particular, the peptide and/or the combination and/or the polypeptide or protein and/or the nucleic acid and/or the carrier and/or the solution and/or the pharmaceutical composition may be injected intravenously (i.v.). Preferably, an RBC or a population of RBCs is coupled to the at least one peptide and/or combination and/or polypeptide or protein and/or contains the nucleic acid and is injected intravenously.

These administration routes are preferred because they are considered tolerogenic. A carrier can also be tolerogenic, for example an RBC or a population of RBCs to which the peptide and/or the combination and/or the polypeptide or protein is coupled or which contains the nucleic acid. The RBC may be part of a solution and/or pharmaceutical composition. Tolerogenic preferably means that the route of administration eventually leads to immune tolerance in the treated subject with respect to the antigens used.

The carrier may be an autologous or an allogeneic cell or a population of autologous or allogeneic cells, preferably an autologous cell or a population of autologous cells, more preferably an autologous RBC or a population of autologous RBCs.

In one embodiment, the carrier in a tolerization approach is an RBC or a population of RBCs and the RBC or the population of RBCs are/is injected intravenously (i.v.) and a single injection dose ranges from 1×10⁷ to 1×10¹² RBCs/kg, preferably 1×10⁹ to 1×10¹¹ RBCs/kg, more preferably 3×10⁹ to 5×10¹⁰ RBCs/kg, more preferably 3×10⁹ to 1×10¹⁰ RBCs/kg. In a preferred embodiment, an amount of 1×10¹¹ to 6×10¹¹ RBCs (which equals 1.7×10⁹ to 1×10¹⁰ RBCs/kg in a 60 kg subject), preferably 2×10¹¹ to 4×10¹¹ RBCs (which equals 3.3×10⁹ to 6.7×10⁹ RBCs/kg in a 60 kg subject), in particular 3×10¹¹ RBCs (which equals 5×10⁹ RBCs/kg in a 60 kg subject), for example in 150 ml saline, or 6×10¹¹ RBCs (which equals 1×10¹⁰ RBCs/kg in a 60 kg subject), for example in 150 ml saline, are used as a single injection dose. The volume to be injected may for example be between 30 and 150 ml, for example 50 ml, 100 ml or 150 ml.

In one embodiment, a single dose is administered in one treatment cycle. In another embodiment, two or more doses are administered with an interval of one week to 6 months, preferably one week to 2 months. Two doses are preferred. In case two doses are taken from the same blood donation, the interval may be limited by the storage life of the RBCs. In that case, the two or more doses are preferably administered within an interval of one week to 2 months. After one treatment cycle the treatment cycle may be repeated. Preferably, there is a period of at least two weeks between treatment cycles.

A tolerization approach may for example be performed as depicted below:

### a) Clinical product

The exemplary clinical product comprises autologous RBCs that have been chemically coupled with the 12 peptides with SEQ ID NOs 1-12 (wherein the peptide with SEQ ID NO: 2 is biotinylated) and re-suspended in saline. The drug product is stored in standard blood bags (Compoflex 1F 600; Fresenius Kabi, Germany) until infusion. A target dose of 3×10¹¹ peptide-coupled RBCs (range 2.2×10¹¹ - 3.5×10¹¹) will be re-suspended in 150 ml saline (NaCl 0.9%).

### b) Autologous RBCs

Autologous RBCs are isolated from whole blood, which is taken from the median cubital vein (or another vein of the arm) of patients, using a standard bag system for blood donations. The separation of RBCs is typically performed by a filtration step to retain leukocytes. Autologous RBCs are stored under standard conditions for RBC products (e.g. erythrocyte concentrates, full blood donation) in blood bags containing stabilizing buffer (e.g. CPDA, SAG-M).

### c) Peptides

The twelve, synthetically manufactured, antigenic peptides are coupled to the surface of RBCs. During manufacturing of the RASGRP2 peptide a Biotin-Polyethylene glycol (PEG)-linker is coupled to the peptide. During manufacturing of biotinRASGRP2 residual amount of Biotin-PEG is removed during the purification steps.

### d) 1-Ethyl-3-(3-dimethylaminopropyl) carbodiimide (EDC)

During the coupling procedure EDC is derivatized to N-Ethyl-N-(dimethylaminopropyl) urea (EDU). Residual EDC and EDU are washed out from the product during the manufacturing procedure.

### e) Composition of unmodified RBCs ("Placebo")

In order to maintain blinding, patients randomized to the two lower dose groups will also receive unmodified autologous RBCs as placebo treatment. The "unmodified" RBCs undergo the same manufacturing procedures, but without the addition of peptides or EDC. Hence the final product comprises autologous RBCs resuspended in saline (same RBC concentration as in the clinical product).

### f) Mode of administration

The clinical product is administered intravenously. Patients are to receive a 150 ml infusion of the clinical product (or placebo, as applicable) within 30 to 60 min.

### g) Outline of the study design

Seven patients will be included in the open-label part A of the trial and sequentially allocated to either the low (single 3×10¹¹ dose; n=2), medium (two doses of 3×10¹¹ administered with a one-week interval; n=2) or high dose (two doses of 6×10¹¹ administered with a one-week interval; n=3). At least 24h must pass between application of the clinical product within the same dose group. Patients, who opted to receive a second cycle will remain in the same dose group, to which they had been allocated in the first cycle.

Part B is a 32-week, multicenter, randomized, parallel-groups, Phase Ila study that will assess safety and efficacy of three doses of the clinical product in subjects with RRMS. The first patients in Part B will be included only after the last patient in Part A has accomplished visit 4, which is the second study drug application within the therapy cycle. In Part B of the study, 45 eligible subjects will be randomized 1:1:1 into 3 treatment groups.

The main efficacy outcome parameter is inflammatory disease activity on brain MRI measured by the number of new/enlarging lesions on T2-weighted images. Since the sensitivity towards inflammatory disease activity in MS is approximately 10-fold higher when using brain MRI as a surrogate for clinical activity (compared to clinical measures such as relapse rate), the direct comparison of a baseline phase with a treatment phase is ideal to follow subtle disease activity and to detect safety concerns at the earliest stage.

In another aspect of the invention, the peptide and/or the combination and/or the polypeptide or protein and/or the nucleic acid and/or the carrier and/or the solution and/or the pharmaceutical composition as disclosed *supra* may be used for identifying a human subject who is suitable for tolerization to autoantigens in MS, preferably early MS, clinically isolated syndrome (CIS), radiologically isolated syndrome (RIS) or relapsing-remitting multiple sclerosis (RRMS).

In another aspect of the invention, a method for identifying a human subject who is suitable for tolerization to autoantigens in MS, preferably early MS, CIS, RIS or RRMS, is provided comprising measuring reactivity of the T cells and/or antibodies against the peptide and/or the combination and/or the polypeptide or protein, which T cells and/or antibodies have been previously obtained from blood, CSF or other body fluid of the subject. Preferably, the method is performed *in vitro.* "Suitable for tolerization" preferably means to identify those patients who would particularly likely profit from antigen-specific tolerance induction, thereby allowing personalizing antigen-specific tolerance approaches.

A method for identifying a human subject who is suitable for tolerization to autoantigens in MS, preferably early MS, CIS, RIS or RRMS is also provided comprising administering the peptide and/or the combination and/or the polypeptide or protein and/or the nucleic acid and/or the carrier and/or the solution and/or the pharmaceutical composition according to the present invention.

Use of the peptide and/or the combination and/or the polypeptide or protein and/or the nucleic acid and/or the carrier and/or the solution and/or the pharmaceutical composition according to the present invention for the manufacture of a medicament for identifying a human subject who is suitable for tolerization to autoantigens in MS, preferably early MS, CIS, RIS or RRMS.

A peptide or polypeptide or protein as defined by any one of SEQ ID NOs: 1 to 37 is preferred, in particular any one of SEQ ID NOs: 1 to 12.

Identifying a human subject, who is suitable for tolerization to autoantigens in MS, preferably early MS, CIS, RIS or RRMS preferably comprises measuring positive reactivity of the T cells and/or antibodies to the peptides (i.e. the peptide preferably being a fragment, i.e. a peptide, of the autoantigen) or polypeptides or proteins in the human subject. A further selection step could be HLA-class II typing and presence of the MS-associated HLA-DR alleles.

Thereby, the human subject can also be diagnosed with MS. Preferably, the diagnosis is an *in vitro* diagnosis. In other words, the identified peptides, combination of peptides and/or polypeptides or proteins can also be used in the diagnosis, in particular *in vitro* diagnosis, of MS. The invention thus also pertains to a method for diagnosing, preferably *in vitro* diagnosing, MS using the peptide and/or the combination and/or the polypeptide or protein and/or the nucleic acid and/or the carrier and/or the solution and/or the pharmaceutical composition as disclosed *supra.*

*In vitro* diagnosis of MS for example comprises the following steps: isolating T cells, preferably CD4+ T cells, and/or antibodies from blood, CSF or other body fluid of the subject and measuring reactivity of the T cells and/or antibodies against a peptide and/or combination of peptides and/or polypeptide or protein according to the present invention. The person skilled in the art is aware of methods for isolating T cells and/or antibodies from blood, CSF or other body fluid of the subject and measuring reactivity of the T cells and/or antibodies against the peptide and/or combination of peptides and/or polypeptide or protein (cf. also *supra*)*.* A positive reactivity of the T cells, preferably CD4+ T cells, and/or antibodies to the tested peptide and/or combination of peptides and/or polypeptide or protein may indicate that the subject suffers from MS.

The diagnosis can also be combined with clinical and imaging findings, i.e. the MS diagnosis (or one of its forms) according to the state of the art, which is typically made by demonstrating neurological deficits and/or MRI lesions compatible with MS that are disseminated in space and time, in particular according to the revised McDonald criteria. These criteria also allow distinguishing between the different forms and disease activity of MS (Thompson et al., 2018, Lancet Neurol, 17(2):162-173).

*In vivo* diagnosis is also possible. For example, a skin prick test or delayed hypersensitivity testing, both common tests known to the skilled person, may indicate reactivity to autoantigens *in vivo.* A skin prick test checks for immediate reactions, while delayed hypersensitivity testing checks for delayed responses (at least 6 hours, typically within 12 to 24 hours).

In another embodiment, ex *vivo* diagnosis is performed which typically includes testing of material, for example serum, CSF or peripheral blood, directly taken from a subject without further treatment or manipulation of the serum, CSF or peripheral blood. For example, antigen reactivity can be tested in serum, CSF or peripheral blood which has previously been obtained from a subject. If for example isolation and/or expansion of cells is required or any other treatment of the serum, CSF or peripheral blood, the method is considered *in vitro.*

Hence, the present invention also relates to above diagnostic method as an ex *vivo* diagnostic method. In addition, the peptide and/or the combination and/or the polypeptide or protein and/or the nucleic acid and/or the carrier and/or the solution and/or the pharmaceutical composition according to the present invention may be used in a method of *in vivo* diagnosing MS.

In another embodiment, the peptide and/or the combination and/or the polypeptide or protein and/or the nucleic acid and/or the carrier and/or the solution and/or the pharmaceutical composition according to the present invention may be used for distinguishing between MS subgroups. The subgroups may for example be defined by the demyelination pattern in the CNS. The skilled person defines four patterns: pattern I, pattern II, pattern III and pattern IV.

In particular, the peptide and/or the combination and/or the polypeptide or protein and/or the nucleic acid and/or the carrier and/or the solution and/or the pharmaceutical composition according to the present invention may be used for diagnosing pattern I and/or pattern II MS in a human subject.

In pattern I MS, the lesions typically show T cell and macrophage infiltration. In pattern II MS, the scar also usually presents T-cells and macrophages around blood vessels, with preservation of oligodendrocytes, and signs of complement system activation.

Hence, a method for diagnosing pattern I and/or pattern II MS using the peptide and/or the combination and/or the polypeptide or protein and/or the nucleic acid and/or the carrier and/or the solution and/or the pharmaceutical composition according to the present invention is also provided. Preferably, the method is performed *in vitro* or ex *vivo,* more preferably *in vitro.*

In the diagnostic methods, a peptide or polypeptide or protein as defined by any one of SEQ ID NOs: 1 to 37 is preferred, in particular any one of SEQ ID NOs: 1 to 12.

In another aspect of the invention, the peptide and/or the combination and/or the polypeptide or protein and/or the nucleic acid and/or the carrier and/or the solution and/or the pharmaceutical composition according to the present invention is used as medicament. The peptides as defined by any of SEQ ID NOs: 1 to 37, preferably SEQ ID NOs: 1 to 12, are preferred.

In another aspect of the invention, a method of controlling a quality of a cell is provided, wherein the cell has been treated with a crosslinker and at least one peptide and/or combination and/or polypeptide and/or protein of the present invention and wherein at least one peptide and/or combination and/or polypeptide or protein is coupled with a marker. Controlling the quality preferably comprises detecting the presence of at least one peptide and/or combination and/or polypeptide or protein on the surface of the cell.

The at least one peptide is preferably a peptide of the combination as disclosed *supra.*

Detecting the presence of at least one peptide and/or combination and/or polypeptide or protein on the surface of the cell can for example be performed by coupling the peptide and/or combination and/or polypeptide or protein with a marker, such as biotin, and detecting the marker, for example by using streptavidin. An exemplary method for measuring coupling efficiency is disclosed *infra.*

In another aspect of the invention, a method of pretesting a human subject diagnosed with MS or a human subject at risk to develop MS is provided using the peptide and/or combination and/or polypeptide or protein according to the present invention. Preferably, the method is performed *in vitro.*

Pretesting a patient with a suitable test to assess reactivity against one or more autoantigens preferably allows tailoring the tolerizing treatment (e.g. composition of the peptides/polypeptides/proteins used for tolerization) to the individual patient or patient subgroup with the aim to render the tolerization as specific as possible and also to avoid potential adverse effects. Antigen-specific tolerization, however, can also be performed in patients in whom a T cell response to the tolerizing antigen has not been shown.

In another aspect of the invention, a method is provided for obtaining CD169+ monocytes which comprises *in vitro* treating monocytes with IFN-α. Thereby, monocytes with no or little CD169 on their surface are induced to express CD169 or more CD169 already after 24 hours. The usual time for obtaining CD169+ monocytes (7 days) is thus shortened. In addition, death and loss of cells can be avoided.

In yet another aspect of the invention, CD169+ monocytes are provided for use in the treatment and/or prevention of multiple sclerosis (MS). In a preferred embodiment, CD169+ monocytes are provided that present immunodominant peptides. An immunodominant peptide preferably is an immunodominant fragment of GDP-L-fucose synthase or a protein of the RASGRP protein family (or an immunodominant derivative or splice variant thereof), such as the peptide and/or the combination and/or the polypeptide or protein of the present invention.

Preferably, an agent is used for the treatment and/or prevention of MS for increasing the CD169 expression on monocytes. Thus, in this aspect of the invention a method of treatment and/or prevention of MS is provided that includes or relies on the increase of CD169 expression on monocytes, in particular on monocytes presenting immunodominant peptides. The increase can have various reasons, e.g., the induction of CD169 expression on the monocytes or the proliferation of CD169+ monocytes.

CD169+ monocytes can also be obtained *in vitro* such as disclosed *supra* by treating monocytes with IFN-α.

An increase in CD169+ monocytes preferably has the effect of an increase in IL-10 in a body fluid, such as blood or CSF.

The agent preferably is a carrier comprising GDP-L-fucose synthase protein or a protein of the RASGRP protein family (or an immunodominant fragment, derivative or splice variant thereof), such as the peptide and/or the combination and/or the polypeptide or protein of the present invention. Most preferably the agent is a carrier, preferably a red blood cell, coupled to an immunodominant peptide or polypeptide or protein or combination as described in the present disclosure.

In another preferred embodiment, the agent is a type-I interferon, for example IFN-α or IFN-β, preferably IFN-α.

In another aspect of the invention, the agent is at least one immunodominant peptide or polypeptide or protein or combination as presently disclosed. This agent is for use in the treatment and/or prevention of MS, wherein the CD169 expression on monocytes is increased upon administration of the immunodominant peptide or polypeptide or protein or combination.

Preferably, the increase is measurable already after 24 h to 48 h.

The CD169+ monocytes and/or the agent are preferably administered into the bloodstream. The monocytes are contacted with the agent in any form that is suitable to induce or maintain CD169 expression. For example, the contact can be *in vivo, in vitro* or ex *vivo.* In one embodiment, CD169+ monocytes are obtained *in vitro* and then administered into a human subject, either together with the agent or before applying the agent, for example applying the agent 1 to 6 hours after the CD169+ monocytes. In another embodiment, monocytes are contacted with the agent *in vitro* (leading to an increase of CD169 expression on the monocytes); after that the CD169+ monocytes are applied to a human subject. In case the agent is RBCs coupled with an immunodominant peptide or polypeptide or protein or combination (for example as presently disclosed), the administered CD169+ monocytes will have preferably processed the peptide or polypeptide or protein in this embodiment. In another embodiment, monocytes are administered together with a type-1 interferon, such as IFN-α or IFN-β and then the agent is administered. Preferably, the agent is an RBC coupled to an immunodominant peptide or polypeptide or protein or combination as presently disclosed. It is also possible to use more than one, i.e. different agents.

Treatment and/or prevention preferably comprises inducing antigen-specific tolerance to autoantigens in a human subject suffering from or at risk of developing MS, in particular inducing antigen-specific tolerance to autoantigens in early MS, CIS, RIS or early RRMS.

CD169+ monocytes are also provided for use in the diagnosis of MS. Preferably, the method of diagnosis is performed *in vitro.*

A method of detecting a tolerogenic response (i. e. the successful induction of tolerance to autoantigens) is also provided comprising measuring the amount of CD169+ monocytes in a human subject that has been treated with a GDP-L-fucose synthase protein or a protein of the RASGRP protein family, or an immunodominant fragment, immunodominant derivative or immunodominant splice variant thereof, or a nucleotide sequence encoding any of the proteins or immunodominant fragment, immunodominant derivative or immunodominant splice variant thereof (or a carrier comprising the same), preferably the peptide and/or the combination and/or the polypeptide or protein and/or the nucleic acid and/or the carrier and/or the pharmaceutical composition of the present invention. An increase of CD169+ monocytes in a human subject indicates a positive tolerogenic response. The method may be *in vitro, in vivo* or ex *vivo.*

Measuring the amount of CD169+ monocytes can be performed by any conventional means known to the person skilled in the art, for example by FACS.

### EXAMPLES

### 1. Identification and solubility of peptides

Based on immunodominant peptides of the proteins TSTA-3 and RASGRP2 previously identified (EP 3 813 865), peptides with different length and sequence were tested for solubility in saline. MBP, MOG and PLP peptides were also tested.

RASGRP2 (74-91) (SEQ ID NO: 2) proved to be soluble in saline at a concentration of 10 mg/ml. This was surprising because the shorter peptide RASGRP2 78-87 (SEQ ID NO: 22) was not soluble in saline. Shorter peptides are usually more soluble than longer peptides. Peptides RASGRP2 70-95 (SEQ ID NO: 23), RASGRP2 78-95 (SEQ ID NO: 24), RASGRP2 68-97 (SEQ ID NO: 25) and RASGRP2 78-92 (SEQ ID NO: 26) were also shown to be insoluble in saline. TSTA-3 peptides with SEQ ID NOs: 27-32 were also shown to be insoluble in saline.

TSTA-3 peptides with SEQ ID NOs: 1, 10, 11 and 12, MBP peptides with SEQ ID NOs: 3-6, MOG peptides with SEQ ID NOs: 7-8 and PLP peptide with SEQ ID NO: 9 were shown to be soluble in saline (10 mg/ml).

### 2. Influence of peptide concentration on coupling efficiency

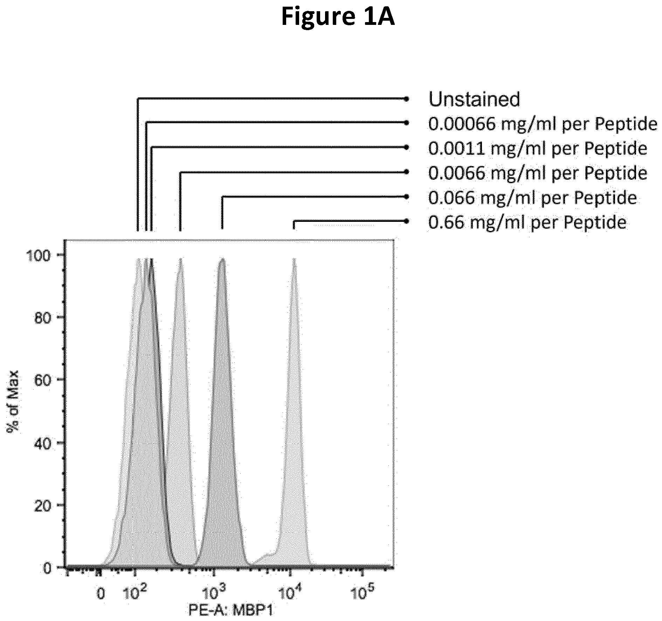

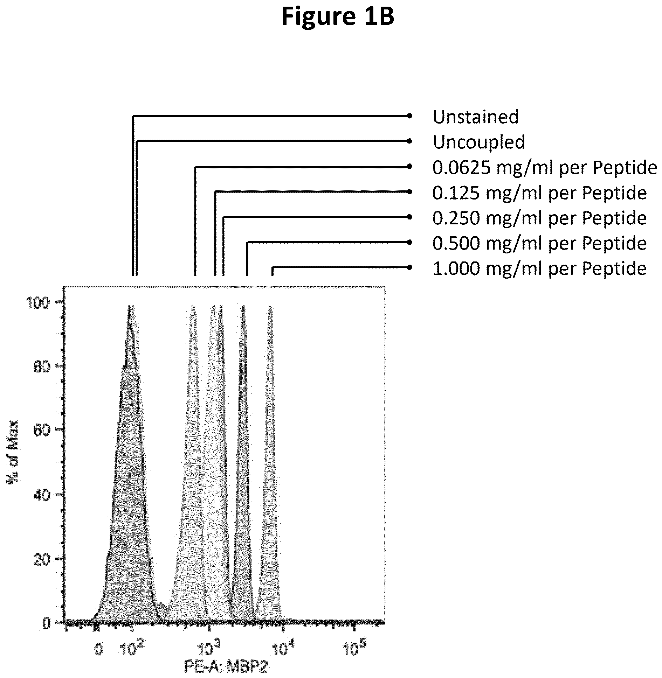

It was tested whether the peptide concentration of each peptide has an influence on coupling efficiency of RBCs. The peptides were used at concentrations of 0.00066 to 0.66 mg/ml (Figure 1A) and 0.0625 to 1 mg/ml (Figure 1B) per peptide. The following peptides were coupled: MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8) and PLP1 (139-154) (SEQ ID NO:9), wherein in the experiment shown in Figure 1A MBP1 was biotinylated and in the experiment shown in Figure 1B MBP2 was biotinylated.

Figure 1 shows that coupling can be performed with a large range of peptide concentrations, in particular above 0.0011 mg/ml per peptide, with a tendency to a more efficient coupling at higher concentrations.

### 3. Influence of EDC concentration on coupling efficiency

It was tested whether the EDC concentration has an influence on coupling efficiency of RBCs. EDC was used at concentrations of 0.5 mg/ml, 1 mg/ml, 3 mg/ml, 5 mg/ml and 10 mg/ml. The following peptides were coupled: MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8) and PLP1 (139-154) (SEQ ID NO:9), wherein MBP4 was biotinylated.

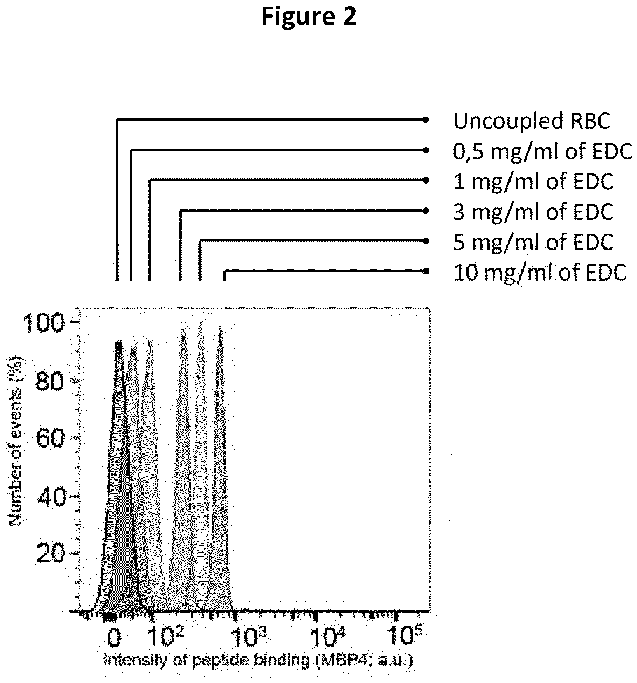

Figure 2 shows that a wide range of EDC concentrations is suitable in a coupling reaction, in particular 3 mg/ml, 5 mg/ml and 10 mg/ml. 30 mg/ml were also tested; there was no difference between 10 mg/ml and 30 mg/ml, probably due to saturation.

### 4. Coupling efficiency of peptides

The 12 peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12) were tested for their coupling efficiency to red blood cells.

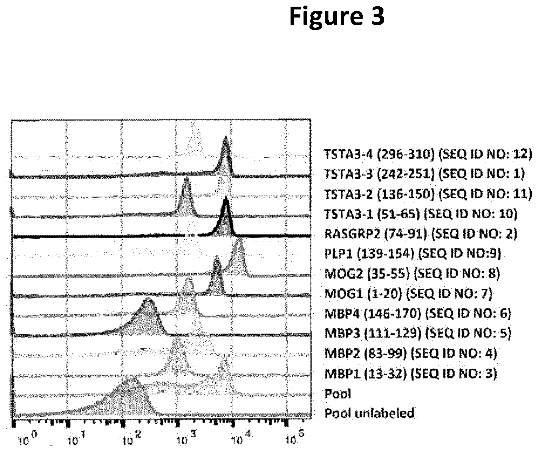

Each of the peptides were biotinylated for coupling detection. Results are shown in Figure 3.

Coupling can be shown for all 12 peptides and for the mixture of all 12 peptides, compared with "Pool unlabeled" which is a mixture of all 12 peptides without biotinylation.

Biotinylation advantageously allowed detection of coupling.

Using confocal microscopy it was shown that coupled peptides are evenly distributed on the cell surface of RBCs (data not shown).

### 5. Biotinylation at the side chain vs. the N-terminus

In this example, biotinylation at the side chain of the cysteine of RASGRP2 (74-91) (SEQ ID NO: 2) was compared with biotinylation at the N-terminus of RASGRP2 (74-91) (SEQ ID NO: 2) with respect to coupling efficiency.

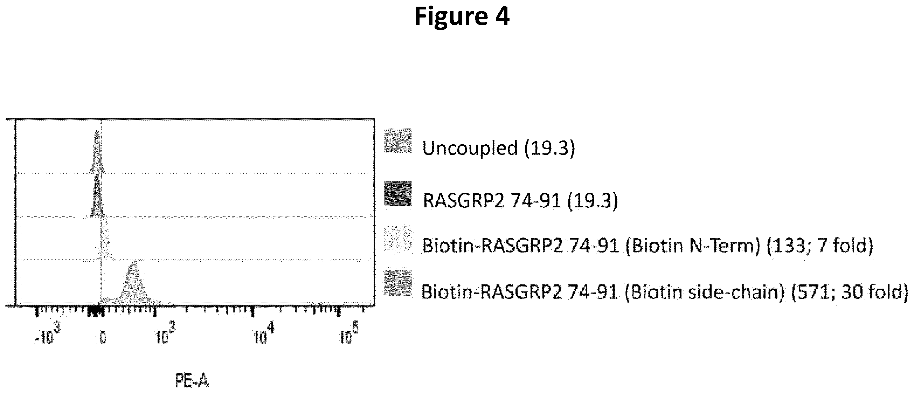

As can be seen in Figure 4, coupling was more efficient when RASGRP2 (74-91) (SEQ ID NO: 2) was biotinylated at the side chain of the cysteine of the peptide.

### 6. T cell reactivity measured in PBMCs

It was tested whether T cell reactivity (secretion of IFN-γ) was measured in PBMCs in response to the peptide RASGRP2 (78-87) (SEQ ID NO: 22), biotinylated RASGRP2 (74-91) (SEQ ID NO: 2), RASGRP2 (74-91) (SEQ ID NO: 2), MOG2 (35-55) (SEQ ID NO: 8) and a negative control (Beads). PBMCs were from a donor with MS and chosen based on prior data showing reactivity of CD45RA⁻ PBMCs to the RASGRP2 (74-91) (SEQ ID NO: 2) peptide.

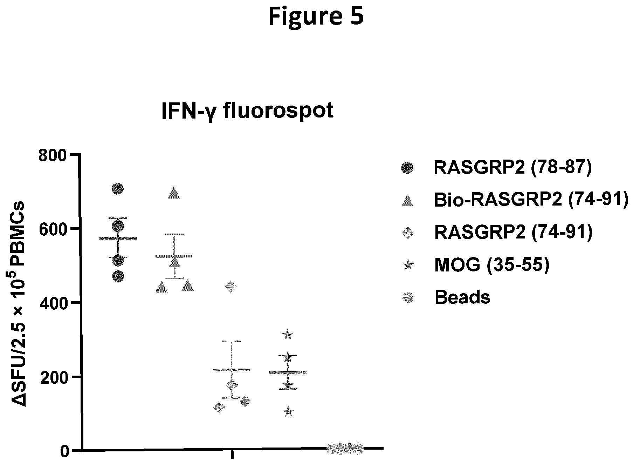

Figure 5 shows IFN-γ secretion in all samples. Hence, PBMCs reacted to all stimuli irrespective of biotinylation.

### 7. T cell reactivity measured in RASGRP2 (78-87) specific T cell clones

Four RASGRP2 78-87-specific T cell clones (TCCs) (cf. Wang et al., 2020, Cell, 183(5):1264-1281) were tested by 3H-thymidine incorporation (72 hours) for their responses against the biotinylated RASGRP2 (74-91) (SEQ ID NO: 2) peptide "bioRASGRP2 74-91" and a positive control, i.e. stimulation with anti-CD2/3/28-coupled beads. Each of the clones is DRB1*15:01-restricted.

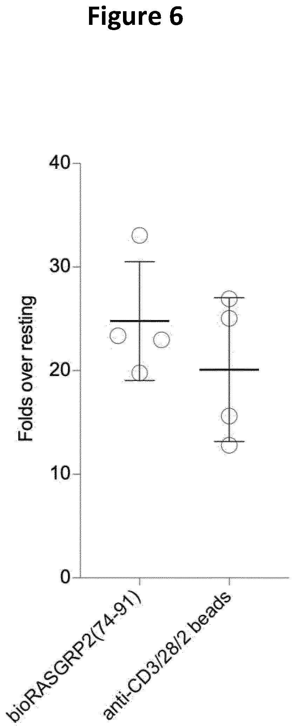

Figure 6 shows a positive proliferative response to the biotinylated RASGRP2 (74-91) (SEQ ID NO: 2) peptide, i.e. T cells specific for RASGRP2 78-87 also recognize the biotinylated RASGRP2 (74-91) (SEQ ID NO: 2) peptide.

### 8. In-vivo and ex-vivo biodistribution of dye-coupled red blood cells in mice

The biodistribution of dye-coupled RBCs following intravenous injection was assessed both *in-vivo* and *ex-vivo.*

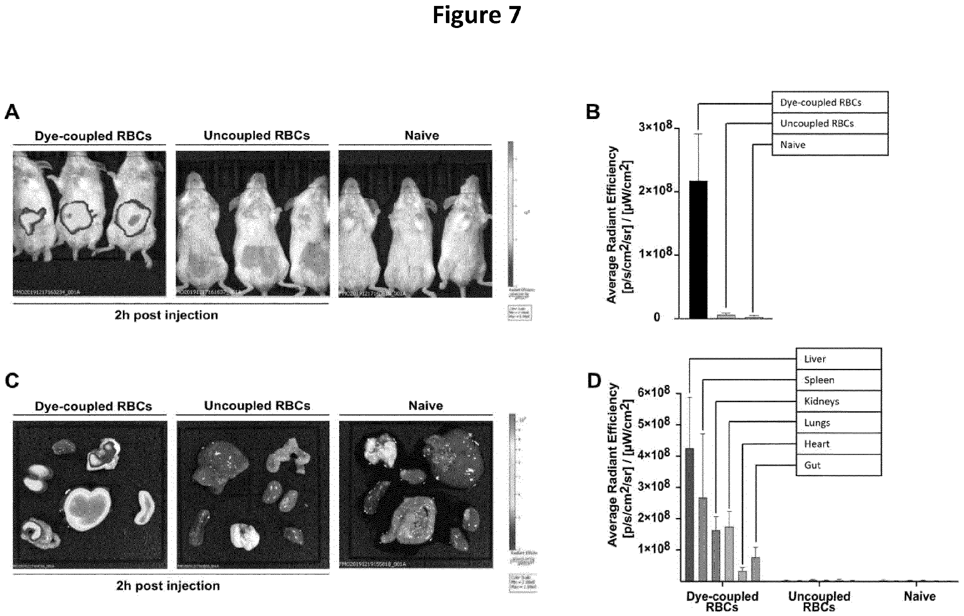

Figure 7A shows whole body imaging 90 minutes post injection. Animals receiving dye-coupled RBCs show a strong signal over the abdomen, compared to control mice. Figure 7B is a graph representing the average radiant efficiency of the three images showing the predominance of the signal in mice receiving the dye-coupled RBCs.

Figure 7C is an *ex-vivo* imaging of single organs confirming the increased recruitment of RBCs coupled with sulfo-Cy7 in the liver, spleen, kidneys and lungs. *Ex-vivo* imaging of single organs confirmed the increased recruitment of RBCs coupled with sulfo-Cy7 in the liver, spleen, kidneys and lungs (Figure 7D).

### 9. Ex-vivo histological analysis of organs

The *in-vivo* findings on the biodistribution were confirmed by *ex-vivo* histological analysis of the organs. Red blood cells were coupled with FAM-labelled PLP1 (139-154) (SEQ ID NO:9) and the cell product injected via the tail vain into mice. The organs liver, spleen, kidney, heart, gut and lungs were removed for histological analysis 2h, 4h, 24h and 48 h after injection.

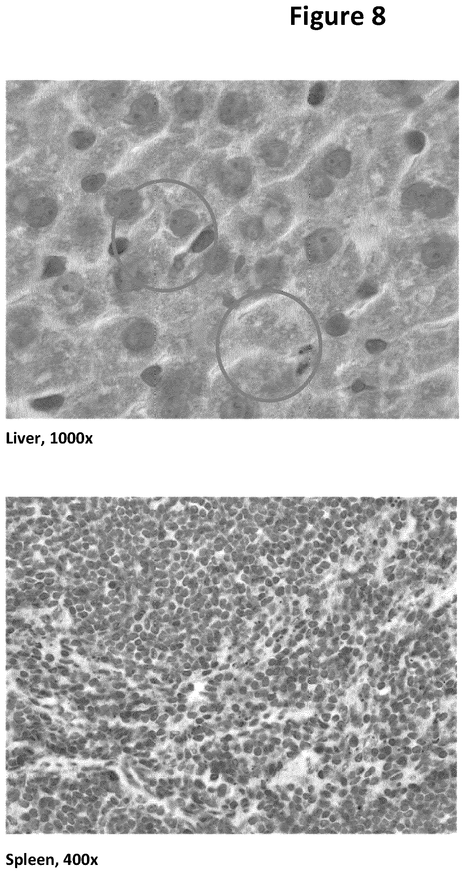

Figure 8 shows FAM-staining of liver and spleen, respectively. Peptide-coupled red blood cells were clearly detected in the liver and spleen as early as 2 hours after injection. 48 h after injection no peptide-coupled RBCs could be detected any more. Mainly the liver and spleen were targeted.

5FAM-peptide-coupled RBCs were detected within F4/80 positive Kupffer cells in the liver and marginal zone macrophages in the spleen. These Kupffer cells and marginal zone macrophages express the scavenger receptor MARCO (data not shown).

### 10. In-vivo biodistribution of peptide-coupled RBCs in MS patients

To determine the distribution and sequestration of peptide-coupled RBCs following infusion in MS patients, the clinically approved labelling technique with ^{99m}Technetium (^{99m}Tc; *Ultra Tag)* was used. Briefly, ^{99m}Tc diffuses into red blood cells, where it gets reduced by stannous ions and binds to hemoglobin to remain inside the RBC.

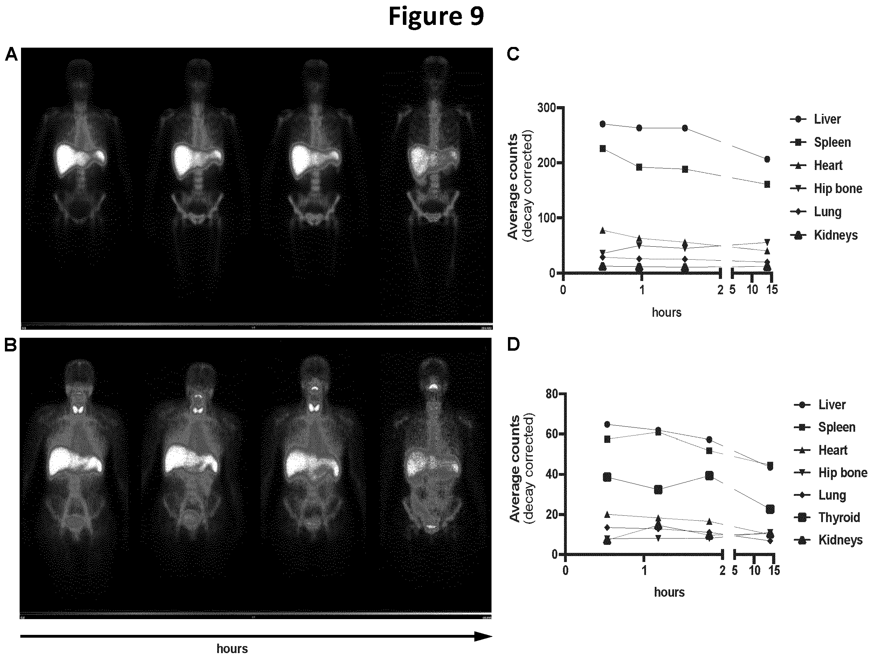

A clear signal could be detected in liver and spleen indicating a removal of the peptide-coupled RBCs in both organs (Figure 9).

Figure 9A and 9B show uptake of peptide-coupled RBCs in the liver and spleen of two individuals by SPECT imaging. Figure 9C and 9D show average counts measured in different organs in the first 15 hours after administration.

### 11. CD169 expression after stimulation of monocytes with IFN-α

In order to shorten the usual time of obtaining CD169+ monocytes (7 days) as well as avoiding their death and losing cells, it was tested whether adding IFN-α to the culture induces the expression of CD169 on monocytes.

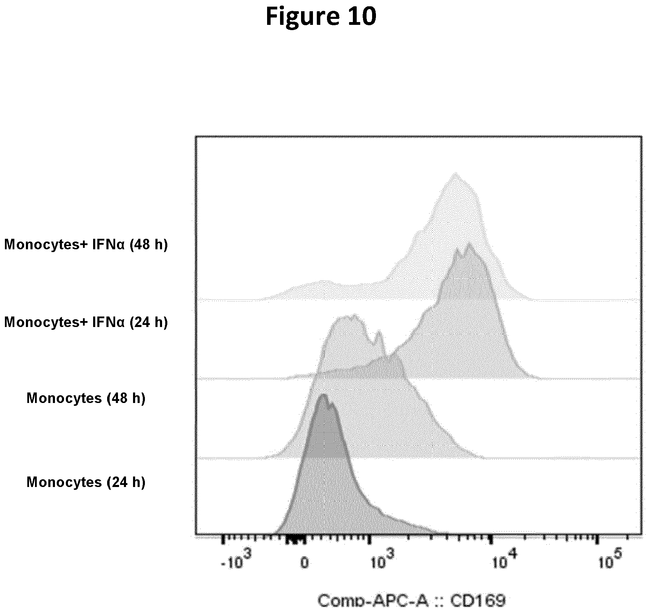

Surprisingly, it was shown that monocytes that were cultured in cRPMI and IFN-α highly express the surface marker CD169 already at 24 hours compared to control (Figure 10). This method significantly decreases the incubation time to obtain CD169+ monocytes while keeping the cells in good quality (almost 100% cells are alive at 24 hours). Furthermore, it does not affect the HLA-DR expression of monocytes.

### 12. CFSE labeling of red blood cells (RBCs) and RBCs coupled to biotinylated RASGRP2 peptide

In order to be able to trace RBCs in co-culture experiments with other cells and to detect whether RBCs are erythrophagocytosed, first the efficiency of CFSE labeling of RBC and RBCs coupled to biotinylated RASGRP2 peptide was tested.

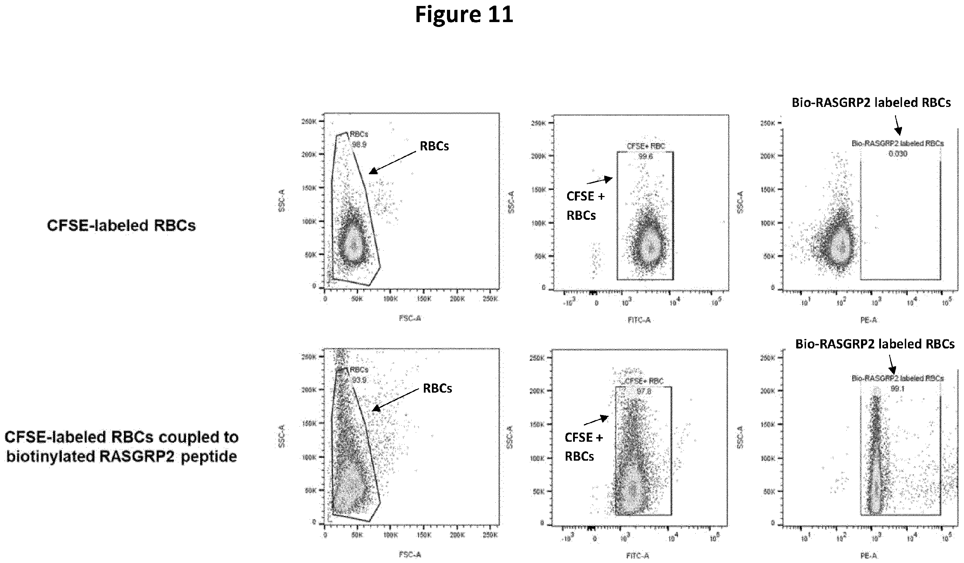

As shown in Figure 11, both RBCs and RBC-EDC-bioRASGRP2 (RBCs coupled to biotinylated RASGRP2 peptide (SEQ ID NO: 2)) are CFSE positive and only the RBC-EDC-bioRASGRP2 is positive for Streptavidin-PE. Hence, CFSE labeling of RBCs is efficient and the coupling is successful.

### 13. Presentation of RASGRP2 peptide by CD169+ monocytes

In order to find out whether erythrophagocytosis by active monocytes leads to processing and further presentation of processed antigens on their surface, the below experiments were designed:
First, monocytes were magnetically isolated from PBMCs and treated with IFN-α (as described *supra*) for 24 hours.
Second, CFSE-labeled RBCs and RBC-EDC-bioRASGRP2 were incubated with active monocytes (CD169+) for 24 and 48 hours (only 24 hours are shown).

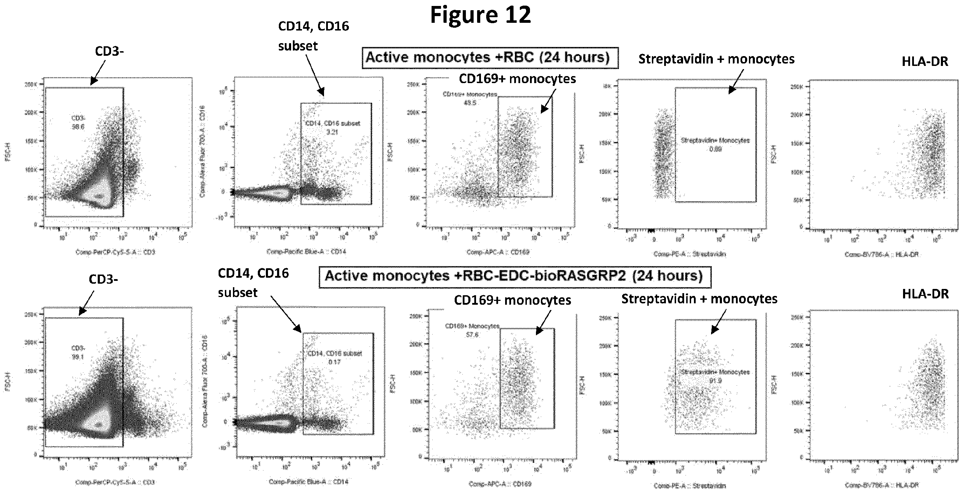

In Figure 12 it is shown that CD3-CD14+CD169+ monocytes are positive for Streptavidin-PE upon incubation with RBC-EDC-bioRASGRP2 (RBCs coupled with biotinylated RASGRP2 peptide) indicating that RBCs coupled with the biotinylated peptide were engulfed, processed and the antigenic peptide presented on the surface of the monocytes. In contrast, no Streptavidin is detected upon incubation with uncoupled RBCs.

### 14. Secretion of IL-10 and IFN-γ upon co-cultivation of TCC14, monocytes and peptide-coupled RBCs

Based on these findings, it was studied whether processing and presentation of peptide-coupled RBCs via CD169+ monocytes leads to activation/stimulation of T cells and whether such stimulation can be detected at 24 or 48 hours.

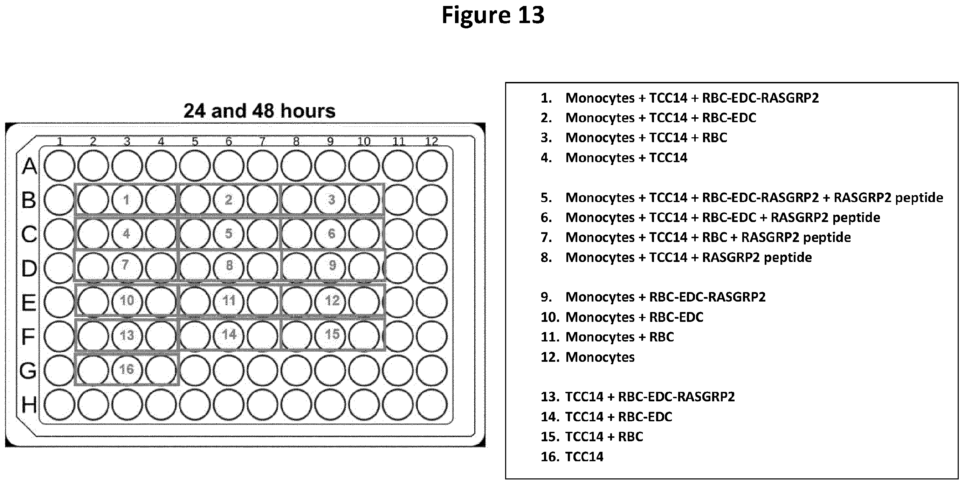

By co-cultivation of CD169+ monocytes that had engulfed peptide-coupled RBCs with T cell clone TCC14, which recognizes RASGRP2 78-87 and had been isolated from the brain of an MS patient (Jelcic et al., 2018) and characterized in detail further by Wang et al. (2020), it was tested if the interaction leads to tolerogenic effects. The plate setup is shown in Figure 13. The experiment was conducted using fluorospot (IFN-γ/IL-10/IL-17) as a functional readout.

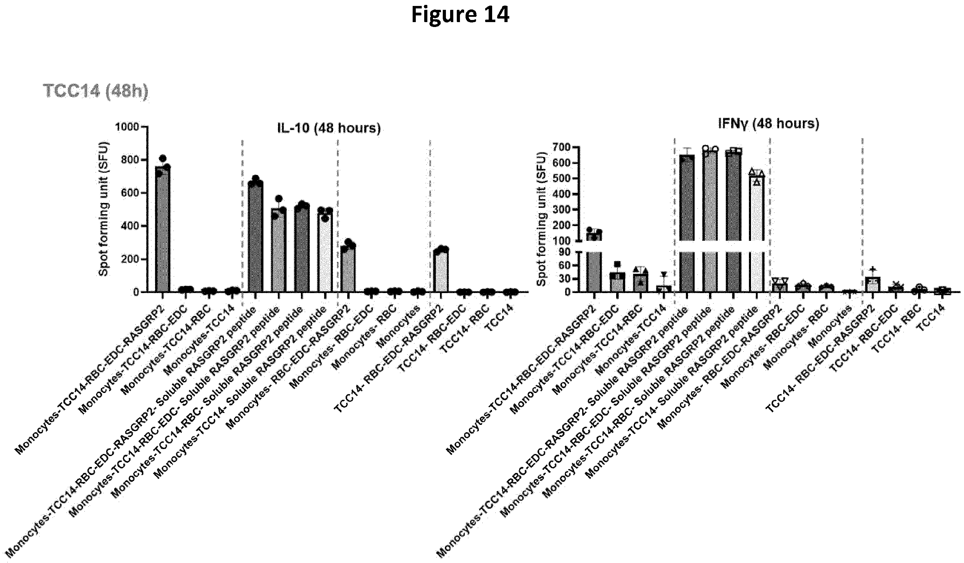

Although TCC14 usually does not secrete IL-10 (Wang et al., 2020), it has been surprisingly shown in Figure 14 that upon cultivation with active monocytes and RASGRP2-coupled RBCs, TCC14 secretes IL-10 (and IFN-γ). Without the antigenic peptide, no IL-10 and almost no IFN-γ was detected.

There is also a robust secretion of IL-10 in any sample with soluble RASGRP2 peptide.

### 15. Antigen specificity of TCC14

In order to evaluate whether the IL-10 and IFN-γ secretion upon co-culturing TCC14 with active monocytes and peptide-coupled RBCs is antigen-specific, biotinylated SIN3A* peptide (SLQNNQPVEFNHAIHYVNKIKNRF) (SEQ ID NO: 38) was coupled to RBCs and co-cultured with TCC14 and active monocytes. TCC14 (an MS autoreactive TCC) does not recognize SIN3A* (a tumor-specific antigen). Therefore, it was tested if the SIN3A*-coupled RBCs have any effect on TCC14. Soluble SIN3A* peptide (QPVEFNHAIHYVN) (SEQ ID NO: 39) was used for stimulation.

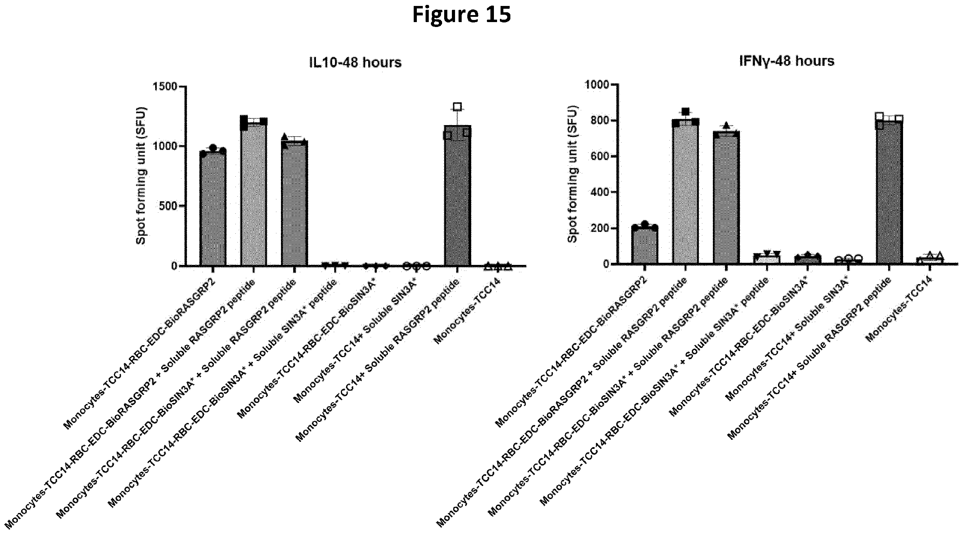

Interestingly, Figure 15 shows that only in the presence of RASGRP2 peptide, either coupled to RBCs or as an exogenous peptide, there is secretion of IL-10 and IFN-γ. These results confirm the previous findings and show that such response is antigen-specific. Using an irrelevant peptide (in this case, SIN3A*) is neither stimulatory nor inhibitory for the T cell clone.

Furthermore, SIN3A* coupled RBCs in the presence of soluble RASGRP2 peptide does not have a positive or negative effect on the T cell response.

### 16. Intracellular cytokine staining of CD4+ cells

To address whether the IL-10 secretion is derived from CD169+ monocytes and/or TCCs, the co-culture experiment with TCC14 was repeated in a U-bottom 96 well plate for 48 hours and the cells were collected for further staining using CD3, CD4, CD14, CD16, CD169, HLA-DR, IFNγ, IL-10 and TGF-β.

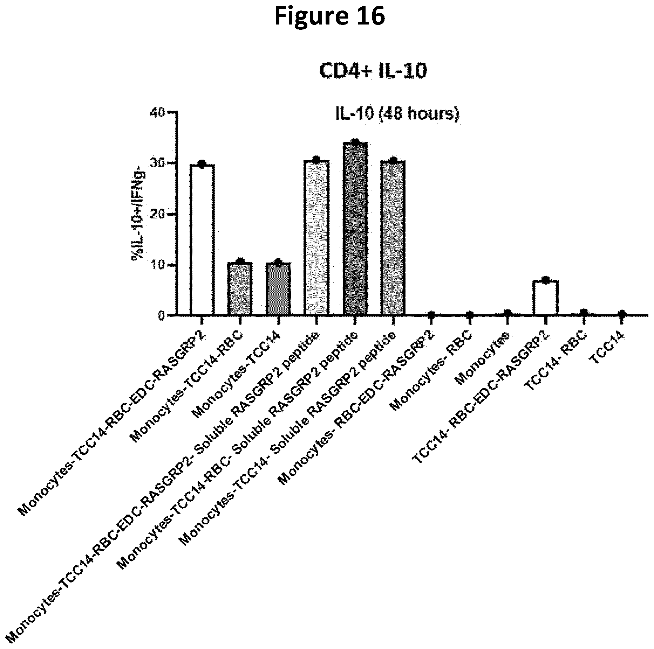

The results from FACS staining confirmed the fluorospot data in that CD4+ T cells appear to be responsible for IL-10 and IFN-γ production (Figure 16). No IL-10 was detected when TCC14 was not present in the sample.

### 17. Materials and Methods

### Synthesis of peptides

All peptides are synthesized on 2-Chlorotrytil resins applying a solid-phase synthesis manufacturing approach commonly used for synthetic peptide manufacture. The introduction of the first amino acid on the resin is achieved through esterification using Ethyldiisopropylamine (EDIA) and the Fluorenylmethyloxycarbonyl (Fmoc) protected amino acid and methanol as capping agent.

Every subsequent coupling of an Fmoc amino acid onto a resin bound amino acid comprises the following steps:
▪ Washing with Dimethylformamide (DMF) for resin conditioning
▪ Coupling the active Esther obtained by dissolving the amino acid, the (Hydroxybenxotriazole) HOBT and Diisopropylcarbodiimide (DIPCDI) in the required amount of DMF
▪ In process control by Kaiser test and reactivating or recoupling if needed
▪ Washing with DMF
▪ Deprotection of the Fmoc group by reacting first for two minutes with 20% Piperidine in DMF, washing the reagents off from the polymeric support and further treating for 10 minutes with fresh 20% Piperidine in DMF
▪ Washing four times with DMF

For the last amino acid cycle that will yield the peptide resin intermediate there were additional washings with DCM, MeOH and Et2O and a step of resin drying. The side chain deprotection of the products was done by treating the peptide resin with 10 mL/gr of peptide resin of a solution containing Trifluoroacetic acid (TFA)/ Thioanisole/Ethanedithiol (EDT)/Phenol/Water (82.5:5:2.5:5:5, v/v/v/v/v). After reacting for 3 hours, the solution was dropped on to 12 mL of cold dry Diethyl ether per mL of cocktail and further six additional washing with 0.5 Liter of diethyl ether were done to the product. Product was dried under vacuum.

The crudes are tested for purity by HPLC and further purified through preparative HPLC in columns loaded with Kromasil C8 of 10 µm in a Dynamic Axially Compressed (DAC) column of 8 cm diameter. Usual flows are in the range of 75-150 ml/min and the detection is done at 220 nm.

For TSTA3 (51-65), TSTA3 (136-150), TSTA3 (296-310) and RASGRP2 (74-91) the fractions collected from the TFA purification, while for TSTA3(242-251) ammonium acetate pH-4.5 purification was performed followed by concentration on preparative HPLC using 0.1% AcOH:I (50:50) buffer eluted fractions are tested by HPLC for purity and the approved fractions are tested for purity and then freeze dried to obtain the peptide before final filtration.

The peptide is then again tested for purity and is dissolved in 0.1N Acetic acid (AcOH)/water (40ml per gram) and Dowex treatment carried out (15min*2cycle) filtered through Polyethersulfone (PES) membranes of 0.45+0.2 µm and submitted to final freeze drying.

### Biotinylation of RASGRP2 Peptide

Peptide RASGRP2 (TFA salt) powder was dissolved in Milli-Q water (1mg/ml in Milli-Q water) until a clear solution was observed. In another container Maleimide (EZ-Link^{®} Maleimide-PEG2-Biotin, Thermo Fisher) reagent (1.3 equivalence) was dissolved in Milli-Q water (1mg per ml in Milli-Q water). Maleimide solution was portion-wise added to the RASGRP2 solution.

The reaction progress was checked using Ellman's Test and HPLC (Limit for RASGRP2 TFA salt: NMT 2.0%). If starting material (RASGRP2 peptide) is below 2.0%, 5% of Acetonitrile is added to the reaction mixture to proceed for TFA purification.

The biotinylated RASGRP2 peptide (biotinRASGRP2) solution obtained is filtered through 0.22 µm membrane filter capsules and collected in a glass beaker in the clean room under a laminar air flow station. After filtration of the solution, the peptide solution is TFA purified and further purified through preparative HPLC in columns loaded with Kromasil C8,10µ silica in a Dynamic Axially Compressed (DAC) column of 8 cm diameter. Usual flowrate is in the range of 75-150 ml/min and the detection is done at 220 nm. The fractions collected from the purification are tested by HPLC for purity.

The biotinRASGRP2 peptide is then again tested for purity and filtered through Polyethersulfone (PES) membranes of 0.22 µm and submitted to final freeze drying.

### Peptide solubility test

Peptides were added to saline at different concentrations and it was visually assessed whether the resulting sample was cloudy (peptide insoluble) or clear (peptide soluble).

### Coupling efficiency of peptides

The peptides have been biotinylated as described *supra.* Detection was performed using fluorophore-conjugated strepatividin. Briefly, red blood cells (3×10⁹/ml) were pulsed with the respective peptides or pool of peptides (final concentration of each peptide 0.132 mg/ml) and EDC (final concentration 3 mg/ml), in saline for 1 h at 4°C. After two washing steps cells were stained with fluorophore-conjugated streptavidin and analyzed by flow cytometry.

For the test on the influence of peptide concentration 3×10⁹ RBCs/ml were used and EDC at 3 mg/ml. The peptides were used at concentrations of 0.00066 to 0.66 mg/ml and 0.0625 to 1 mg/ml per peptide, respectively.

For the test on the influence of EDC concentration 2×10⁹ RBCs/ml were used and 0.2 mg/ml per peptide. EDC-HCl was used at concentrations of 0.5 mg/ml, 1 mg/ml, 3 mg/ml, 5 mg/ml or 10 mg/ml.

The comparison of coupling with a peptide biotinylated at a side chain vs. N-terminus has been performed with 3×10⁹ RBCs/ml and EDC-HCl at 3 mg/ml.

### Fluorospot assay

T cell reactivity in PBMCs in response to different peptides (IFN-γ fluorospot assay):
2.5 × 10⁵ PBMCs were seeded in each well in 200 µl X-VIVO. Each peptide had a final concentration of 10 µM per well. The cells were incubated for 44 hours.

Co-cultivation of TCC14, monocytes and peptide-coupled RBCs (IFN-γ/IL-10/IL-17 fluorospot assay):
The plate (setup Figure 13) was washed with PBS (3 times) as instructed by the manufacturer. The blocking was conducted using cRPMI for 30 minutes. 25 ×10³ of the TCCs were seeded in each well and 50×10³ magnetically isolated monocytes were seeded in each well (final volume 200 µl). Monocytes were isolated from autologous PBMCs of an HLA matched donor using CD14 microbeads as instructed by the manufacturer.

For the coupling 1.58 mg/ml (0.132 mg/ml × 12) of the biotinylated RASGRP2 74-91 peptide (SEQ ID NO: 2) was used. 200×10³ RBCs were added to each well.

IFN-α was directly added into the wells at the time of seeding the cells at 10000 U/ml. In the samples with soluble RASGRP2 peptide, 10 µM of non-biotinylated RASGRP2 78-92 (SEQ ID NO: 26) was added to the wells. Plates were incubated for 24 and 48 hours (only the latter shown) and stained for IFNγ, IL-10 and IL-17 according to the manufacturer's instructions.

### 3H-thymidine assay

Proliferation was measured 72 h after stimulation by 3H-thymidine (Hartmann Analytic, Braunschweig, Germany) incorporation in a scintillation counter (Wallac 1450, PerkinElmer, Rodgau-Jürgesheim, Germany). Depicted is the fold change over unstimulated control. Each dot represents the mean of 3 repeat wells for each clone. Peptides were presented in the assay by BLS cells transfected with DRA*01:01 and DRB1*15:01 (the heterodimer is referred to as BLS DR2b).

### In-vivo and ex-vivo biodistribution assay in mice

RBCs were coupled with sulfo-Cy7 and injected into mice. Imaging was performed 90 minutes post injection. Images were acquired with a IVIS Lumina XRMS series III (Perkin Elmer).

### Ex-vivo histological analysis

For the coupling, 0.924 mg/ml peptide (FAM-labelled PLP1 (139-154) (SEQ ID NO:9)) and 3 mg/ml EDC-HCl were used. 1×10⁹ cells were injected per mouse. Staining was performed with an anti-5FAM-HRP antibody.

### In vivo biodistribution in humans

^{99m}Tc-labelled, GMP-manufactured peptide-coupled RBCs were injected intravenously, and the distribution was visualized by rapid and dynamic imaging acquisition via gamma scintigraphy and CT-Single Photon Emission Tomography (SPECT).

### IFN-α stimulation of monocytes and expression of CD169

Human recombinant IFN-α (2A) (Stemcell technologies) was used at 10000 U/ml in the cRPMI medium of freshly isolated CD14+ monocytes and expression of CD169 was measured after 24 and 48 hours compared to the control group (3 × 10⁷ monocytes were seeded in each well of a 6-well plate in cRPMI).

### CFSE labeling of red blood cells (RBCs) and RBCs coupled to biotinylated RASGRP2 peptide

RBCs were coupled with 1.58 mg/ml (0.132 mg/ml × 12) of the biotinylated RASGRP2 74-91 peptide (SEQ ID NO: 2) ("RBC-EDC-bioRASGRP2"). CellTrace^{™} CFSE Cell Proliferation Kit (Catalog number: C34554) was used to label the RBCs (1 µM CFSE and 20 minutes of incubation). Coupling efficiency (by streptavidin-PE) and CFSE labelling was measured using FACS.

### Co-cultivation of RBCs and RBC-EDC-bioRASGRP2 with active monocytes (CD169+)

CFSE-labeled RBCs and RBC-EDC-bioRASGRP2 (RBCs coupled with biotinylated RASGRP2 peptide) were seeded with active monocytes (CD169+) for 24 and 48 hours (only 24 hours are shown) in a 6-well plate in cRPMI medium (3×10⁶ monocytes/well were cultured with 10⁸ RBCs and RBC-EDC-bioRASGRP2).

After 24 and 48 hours cells were harvested and stained with antibodies (only 24 hours are shown). Analysis was performed by FACS.

### Co-cultivation of TCC14, monocytes and peptide-coupled RBCs testing antigen specificity of TCC14

Monocytes were magnetically isolated from PBMCs of a DR15 donor and seeded at 50×10³ in each well. 25 ×10³ of the TCC14 was seeded in each well (final volume 200 µl). Coupling to RBCs was performed with 1.58 mg/ml (0.132 mg/ml × 12) of the respective peptide. 200×10³ RBCs were added to each well.

IFN-α was directly added into the wells at the time of seeding the cells at 10000 U/ml. In the samples with soluble peptides, 10 µM of the peptides was added to the wells. Plates were incubated for 48 hours and stained for IFN-γ, IL-10 and IL-17 according to the manufacturer's instructions (see fluorospot assay *supra).*

### Intracellular cytokine staining of CD4+ cells

Cells (TCC14, monocytes and RBCs as indicated) were incubated for a total of 44 hours. For the last 4 hours, BFA was added to cells (BD GolgiPlug, 1 µl per ml cell culture). Wells were pooled, washed once in PBS and stained in Zombie NIR for 20 minutes.

Next, cells were washed once in FACS buffer and resuspend in 50 µl volume containing 2.5 µl FcX Block and incubated for 10 minutes. 50 µl of surface stain mix was added and incubated for 20 minutes. Cells were then washed in FACS buffer and fix/perm solution was added for 45 minutes. 2 ml perm buffer was added, removed, and intracellular antibodies were added in perm buffer and cells stained for 30 minutes.

The samples were washed twice in perm buffer and resuspended in 200 µl FACS buffer and analyzed by FACS.

### EMBODIMENTS

1. A peptide comprising the sequence of TSTA3-3 (242-251) (SEQ ID NO: 1).
2. A peptide consisting of the sequence of TSTA3-3 (242-251) (SEQ ID NO: 1).
3. A peptide comprising the sequence of RASGRP2 (74-91) (SEQ ID NO: 2).
4. A peptide consisting of the sequence of RASGRP2 (74-91) (SEQ ID NO: 2).
5. A peptide comprising a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1.
   An identity of at least 80 %, more preferably at least 85 %, more preferably at least 90 %, more preferably at least 95 %, more preferably at least 96 %, more preferably at least 97 %, more preferably at least 98 %, more preferably at least 99 % is especially preferred.
6. A peptide consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1.
   An identity of at least 80 %, more preferably at least 85 %, more preferably at least 90 %, more preferably at least 95 %, more preferably at least 96 %, more preferably at least 97 %, more preferably at least 98 %, more preferably at least 99 % is especially preferred.
7. A peptide comprising a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 2.
   An identity of at least 80 %, more preferably at least 85 %, more preferably at least 90 %, more preferably at least 95 %, more preferably at least 96 %, more preferably at least 97 %, more preferably at least 98 %, more preferably at least 99 % is especially preferred.
8. A peptide consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 2.
   An identity of at least 80 %, more preferably at least 85 %, more preferably at least 90 %, more preferably at least 95 %, more preferably at least 96 %, more preferably at least 97 %, more preferably at least 98 %, more preferably at least 99 % is especially preferred.
9. A combination comprising one or more peptides according to any of the preceding embodiments and one or more of the following peptides:
   a) a peptide comprising or consisting of the sequence of MBP1 (13-32) (SEQ ID NO: 3),
   b) a peptide comprising or consisting of the sequence of MBP2 (83-99) (SEQ ID NO: 4),
   c) a peptide comprising or consisting of the sequence of MBP3 (111-129) (SEQ ID NO: 5),
   d) a peptide comprising or consisting of the sequence of MBP4 (146-170) (SEQ ID NO: 6),
   e) a peptide comprising or consisting of the sequence of MOG1 (1-20) (SEQ ID NO: 7),
   f) a peptide comprising or consisting of the sequence of MOG2 (35-55) (SEQ ID NO: 8),
   g) a peptide comprising or consisting of the sequence of PLP1 (139-154) (SEQ ID NO:9),
   h) a peptide comprising or consisting of the sequence of TSTA3-1 (51-65) (SEQ ID NO: 10),
   i) a peptide comprising or consisting of the sequence of TSTA3-2 (136-150) (SEQ ID NO: 11),
   j) a peptide comprising or consisting of the sequence of TSTA3-4 (296-310) (SEQ ID NO: 12), or
   k) a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or SEQ ID NO: 12.
   An identity of at least 80 %, more preferably at least 85 %, more preferably of at least 90 %, more preferably at least 95 %, more preferably at least 96 %, more preferably at least 97 %, more preferably at least 98 %, more preferably at least 99 % is especially preferred.
10. A combination comprising the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12).
11. A combination consisting of the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12).
12. The peptide and/or the combination according to any of the preceding embodiments, wherein the peptide and/or at least one, preferably all peptides of the combination each has/have a maximum length of 50 amino acids.
13. A polypeptide or protein comprising at least two peptide sequences, wherein the at least two peptide sequences are selected from the peptide and/or the combination according to any one of the preceding embodiments.
14. The polypeptide or protein according to embodiment 13, wherein the polypeptide or protein comprises a linker between at least two of the peptide sequences.
15. The polypeptide or protein according to embodiment 13 or 14, wherein the polypeptide or protein comprises 2 or 3 or 4 or 5 or 6 or 7 or 8 or 9 or 10 or 11 or 12 or more different peptide sequences.
   It is especially preferred to link 6 to 12 peptides. It is also possible to incorporate the same peptide more than once.
16. The polypeptide or protein according to embodiment 14 or 15, wherein at least one linker is VVR or GGS, preferably GGS.
17. The polypeptide or protein according to any one of embodiments 13 to 16, comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with any one of the sequences of SEQ ID NOs: 33 to 37.
18. The polypeptide or protein according to any one of embodiments 13 to 16, comprising or consisting of at least one of the sequences as set forth in SEQ ID NOs: 33-37.
19. The polypeptide or protein according to any one of embodiments 13 to 16 comprising or consisting of the sequence as set forth in SEQ ID NO: 37.
20. The peptide and/or the combination and/or the polypeptide or protein according to any of the preceding embodiments, wherein the peptide and/or at least one peptide of the combination and/or the polypeptide or protein comprises a His Tag.
21. The peptide and/or the combination and/or the polypeptide or protein according to embodiment 20, wherein the His Tag comprises two to ten histidine residues.
22. The peptide and/or the combination and/or the polypeptide or protein according to embodiment 20 or 21, wherein the His Tag consists of six histidine residues.
23. The peptide and/or the combination and/or the polypeptide or protein according to any one of embodiments 20 to 22, wherein the His Tag is located at the N-terminus of the peptide and/or polypeptide or protein.
24. The peptide and/or the combination and/or the polypeptide or protein according to any of the preceding embodiments, wherein the peptide and/or at least one, preferably all peptides of the combination and/or the polypeptide or protein is/are soluble in a physiological solution.
   Preferably, the peptide and/or at least one, preferably all peptides of the combination, each has/have a maximum length of 50 amino acids and is/are soluble in a physiological solution.
25. The peptide and/or the combination and/or the polypeptide or protein according to embodiment 24, wherein the physiological solution is isotonic.
26. The peptide and/or the combination and/or the polypeptide or protein according to embodiment 24 or 25, wherein the physiological solution is saline or Ringer's lactate solution, preferably saline.
27. The peptide and/or the combination and/or the polypeptide or protein according to any of the preceding embodiments, wherein the peptide and/or at least one, preferably all peptides of the combination and/or the polypeptide or protein is/are immunodominant.
28. The peptide and/or the combination and/or the polypeptide or protein according to any of the preceding embodiments, wherein at least one peptide and/or polypeptide or protein is modified.
29. The peptide and/or the combination and/or the polypeptide or protein according to embodiment 28, wherein the at least one peptide and/or polypeptide or protein is coupled with a marker.
30. The peptide and/or the combination and/or the polypeptide or protein according to embodiment 29, wherein the marker is biotin or a fluorescent dye, such as FAM or Cy7, preferably biotin.
31. The peptide and/or the combination and/or the polypeptide or protein according to embodiment 29 or 30, wherein the marker, preferably the biotinylation, is within the first 15, preferably within the first 10, more preferably within the first 5 amino acids from the N-terminus and/or within the last 15, preferably within the last 10, more preferably within the last 5 amino acids from the C-terminus.
32. The peptide and/or the combination and/or the polypeptide or protein according to any one of embodiments 29 to 31, wherein the marker is biotin and the biotinylation takes place at a cysteine, preferably at the thiol group of the cysteine.
33. The peptide and/or the combination and/or the polypeptide or protein according to any one of embodiments 29 to 32, wherein the marker is biotin and the biotin is coupled to the at least one peptide and/or polypeptide or protein by a polyethylene glycol (PEG) linker.
34. The peptide and/or the combination and/or the polypeptide or protein according to any one of embodiments 30 to 33, wherein RASGRP2 (74-91) (SEQ ID NO: 2) is biotinylated, preferably on the cysteine.
35. A nucleic acid encoding the peptide and/or the combination and/or the polypeptide or protein according to any one of embodiments 1 to 34.
36. The peptide and/or the combination and/or the polypeptide or protein and/or the nucleic acid according to any one of embodiments 1 to 35, wherein the peptide and/or the combination and/or the polypeptide or protein and/or the nucleic acid is isolated.
37. A carrier comprising the peptide and/or the combination and/or the polypeptide or protein and/or the nucleic acid according to any one of embodiments 1 to 36.
38. The carrier according to embodiment 37, wherein the carrier is adapted to or is capable of carrying or transporting the peptide and/or the combination and/or the polypeptide or protein and/or the nucleic acid according to any one of embodiments 1 to 36.
39. The carrier according to embodiment 37 or 38, wherein the carrier is coupled to the peptide and/or the combination and/or the polypeptide or protein and/or contains the nucleic acid according to any one of embodiments 1 to 36.
40. The carrier according to any one of embodiments 37 to 39, wherein the carrier is selected from the group consisting of a cell or a population of cells, a membrane of a cell, a protein, a lipid, a glycolipid, a bead, a nanoparticle, a virus-like-particle (VLP) and a molecule, such as a sugar molecule, and any combination thereof.
41. The carrier according to any one of embodiments 37 to 40, wherein the carrier is a cell or a population of cells, and the peptide, combination, polypeptide or protein is expressed by the cell or population of cells.
42. The carrier according to embodiment 40 or 41, wherein the cell is a blood cell or the population of cells is a population of blood cells.
43. The carrier according to embodiment 42, wherein the blood cell is a red or white blood cell, preferably a red blood cell (RBC) or the population of cells is a population of red or white blood cells, preferably a population of RBCs.
44. The carrier according to any one of embodiments 37 to 43, wherein the carrier is a cell or population of cells chemically coupled to the peptide and/or the combination and/or the polypeptide or protein according to any one of embodiments 1 to 34 or embodiment 36.
45. The carrier according to embodiment 44, wherein the cell or population of cells is a blood cell or a population of blood cells, respectively.
46. The carrier according to embodiment 45, wherein the blood cell is a red or white blood cell, preferably a red blood cell (RBC), or the population of blood cells is a population of red or white blood cells, preferably a population of red blood cells (RBCs), respectively.
47. The carrier according to any one of embodiments 44 to 46, wherein the carrier is an RBC or a population of RBCs, chemically coupled to at least the peptide TSTA3-3 (242-251) (SEQ ID NO: 1).
48. The carrier according to any one of embodiments 44 to 46, wherein the carrier is an RBC or a population of RBCs, chemically coupled to at least the peptide RASGRP2 (74-91) (SEQ ID NO: 2).
49. The carrier according to any one of embodiments 44 to 46, wherein the carrier is an RBC or a population of RBCs, chemically coupled to a combination comprising the peptide TSTA3-3 (242-251) (SEQ ID NO: 1) and/or the peptide RASGRP2 (74-91) (SEQ ID NO: 2), and at least one peptide selecting from the group consisting of MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12).
50. The carrier according to any one of embodiments 44 to 46, wherein the carrier is an RBC or a population of RBCs, chemically coupled to a combination of peptides comprising or consisting of TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12).
51. The carrier according to any one of embodiments 37 to 50, wherein at least one peptide is biotinylated.
52. The carrier according to embodiment 51, wherein RASGRP2 (74-91) (SEQ ID NO: 2) and/or TSTA3-3 (242-251) (SEQ ID NO: 1) is coupled and biotinylated, preferably wherein RASGRP2 (74-91) (SEQ ID NO: 2) is biotinylated.
53. The carrier according to any one of embodiments 44 to 46, wherein the carrier is an RBC or a population of RBCs, chemically coupled to at least one polypeptide or protein comprising or consisting of a sequence as set forth in any one of SEQ ID NOs: 33-37.
54. The carrier according to any one of embodiments 44 to 46, wherein the carrier is an RBC or a population of RBCs, chemically coupled to at least one polypeptide or protein comprising or consisting of a sequence as set forth in SEQ ID NO: 37.
55. The carrier according to embodiment 53 or 54, wherein at least one polypeptide or protein is biotinylated, preferably wherein the polypeptide or protein comprising or consisting of a sequence as set forth in SEQ ID NO: 37 is biotinylated.
56. The carrier according to any one of embodiments 40 to 55, wherein the cell or population of cells is chemically coupled by 1-ethyl-3-(3-dimethylaminopropyl)-carbodiimide (ECDI/EDC).
57. A solution comprising the peptide and/or the combination and/or the polypeptide or protein and/or the nucleic acid and/or the carrier according to any one of embodiments 1 to 56.
58. The solution according to embodiment 57, wherein the solution is isotonic.
59. The solution according to embodiment 57 or 58, wherein the solution is saline or Ringer's lactate solution, preferably saline.
60. A pharmaceutical composition comprising the peptide and/or the combination and/or the polypeptide or protein and/or the nucleic acid and/or the carrier and/or the solution according to any one of embodiments 1 to 59, and a pharmaceutically acceptable excipient.
61. The peptide and/or the combination and/or the polypeptide or protein and/or the nucleic acid and/or the carrier and/or the solution and/or the pharmaceutical composition according to any one of embodiments 1 to 60 for use in the treatment and/or prevention of multiple sclerosis (MS). Preferably, the carrier is a cell or a population of cells.
62. The peptide and/or the combination and/or the polypeptide or protein and/or the nucleic acid and/or the carrier and/or the solution and/or the pharmaceutical composition according to any one of embodiments 1 to 60 for use in a method for inducing antigen-specific tolerance to autoantigens in a human subject suffering from or at risk of developing MS. Preferably, the carrier is a cell or a population of cells.
63. The peptide and/or the combination and/or the polypeptide or protein and/or the nucleic acid and/or the carrier and/or the solution and/or the pharmaceutical composition according to any one of embodiments 1 to 60 for use in a method for inducing antigen-specific tolerance to autoantigens in a human subject with early MS. Preferably, the carrier is a cell or a population of cells.
64. The peptide and/or the combination and/or the polypeptide or protein and/or the nucleic acid and/or the carrier and/or the solution and/or the pharmaceutical composition for use according to any one of embodiments 61 to 63, wherein the peptide and/or the combination and/or the polypeptide or protein and/or the nucleic acid and/or the carrier and/or the solution and/or the pharmaceutical composition is administered by subcutaneous (s.c.), intracoelomic (i.c), intramuscular (i.m.), intradermal (i.d.), transdermal (t.d.) or intravenous (i.v.) administration. Preferably, the carrier is a cell or a population of cells.
65. The peptide and/or the combination and/or the polypeptide or protein and/or the nucleic acid and/or the carrier and/or the solution and/or the pharmaceutical composition for use according to any one of embodiments 61 to 63, wherein the peptide and/or the combination and/or the polypeptide or protein and/or the solution and/or the pharmaceutical composition is administered by nasal, inhaled or oral administration.
66. The peptide and/or the combination and/or the polypeptide or protein and/or the nucleic acid and/or the carrier and/or the solution and/or the pharmaceutical composition for use according to any one of embodiments 61 to 65, wherein the carrier is an autologous or an allogeneic cell or a population of autologous or allogeneic cells, preferably an autologous cell or a population of autologous cells, more preferably an autologous RBC or a population of autologous RBCs.
67. The peptide and/or the combination and/or the polypeptide or protein and/or the nucleic acid and/or the carrier and/or the solution and/or the pharmaceutical composition for use according to any one of embodiments 61 to 64 or 66, wherein the peptide and/or the combination and/or the polypeptide or protein and/or the nucleic acid and/or the carrier and/or the solution and/or the pharmaceutical composition is injected intravenously (i.v.).
   Preferably, an RBC or a population of RBCs is coupled to the at least one peptide and/or combination and/or polypeptide or protein and/or contains the nucleic acid and is injected intravenously.
68. The peptide and/or the combination and/or the polypeptide or protein and/or the nucleic acid and/or the carrier and/or the solution and/or the pharmaceutical composition for use according to embodiment 67, wherein the carrier is an RBC or a population of RBCs and the RBC or the population of RBCs are/is injected intravenously (i.v.) and a single injection dose ranges from 1×10⁷ to 1×10¹² RBCs/kg, preferably 1×10⁹ to 1×10¹¹ RBCs/kg, more preferably 3×10⁹ to 5×10¹⁰ RBCs/kg, more preferably 3×10⁹ to 1×10¹⁰ RBCs/kg. In a preferred embodiment, an amount of 1×10¹¹ to 6×10¹¹ RBCs, preferably 2×10¹¹ to 4×10¹¹ RBCs, in particular 3×10¹¹ RBCs or 6×10¹¹ RBCs are used as a single injection dose.
69. The peptide and/or the combination and/or the polypeptide or protein and/or the nucleic acid and/or the carrier and/or the solution and/or the pharmaceutical composition for use according to embodiment 68, wherein a single dose is administered in one treatment cycle.
70. The peptide and/or the combination and/or the polypeptide or protein and/or the nucleic acid and/or the carrier and/or the solution and/or the pharmaceutical composition for use according to embodiment 68, wherein two or more doses are administered with an interval of one week to 6 months, preferably one week to 2 months.
71. A method for diagnosing MS using the peptide and/or the combination and/or the polypeptide or protein and/or the nucleic acid and/or the carrier and/or the solution and/or the pharmaceutical composition according to any one of embodiments 1 to 60. Preferably, the method is performed *in vitro* or ex *vivo,* more preferably *in vitro.*
   A peptide or polypeptide or protein as defined by any one of SEQ ID NOs: 1 to 37 is preferred, in particular any one of SEQ ID NOs: 1 to 12.
72. A method for diagnosing pattern I and/or pattern II MS using the peptide and/or the combination and/or the polypeptide or protein and/or the nucleic acid and/or the carrier and/or the solution and/or the pharmaceutical composition according to any one of embodiments 1 to 60.
   Preferably, the method is performed *in vitro* or ex *vivo,* more preferably *in vitro.*
   A peptide or polypeptide or protein as defined by any one of SEQ ID NOs: 1 to 37 is preferred, in particular any one of SEQ ID NOs: 1 to 12. Hence, the peptide and/or the combination and/or the polypeptide or protein and/or the nucleic acid and/or the carrier and/or the solution and/or the pharmaceutical composition can be used in a method for distinguishing between MS subgroups, in particular in a method for diagnosing pattern I and/or pattern II MS in a human subject. Preferably, the method is performed *in vitro.*
73. The method according to embodiment 71 or 72, wherein the method comprises measuring reactivity of T cells and/or antibodies against the peptide and/or the combination and/or the polypeptide or protein according to any one of embodiments 1 to 34 or 36, which T cells and/or antibodies have been previously obtained from blood, CSF or other body fluid of the subject. Preferably, the method is performed *in vitro* or ex *vivo,* more preferably *in vitro.*
74. The peptide and/or the combination and/or the polypeptide or protein and/or the nucleic acid and/or the carrier and/or the solution and/or the pharmaceutical composition according to any one of embodiments 1 to 60 for use in a method of diagnosing MS, in particular pattern I and/or pattern II MS. Preferably, the method is performed *in vivo.* A peptide or polypeptide or protein as defined by any one of SEQ ID NOs: 1 to 37 is preferred, in particular any one of SEQ ID NOs: 1 to 12.
75. The peptide and/or the combination and/or the polypeptide or protein and/or the nucleic acid and/or the carrier and/or the solution and/or the pharmaceutical composition according to any one of embodiments 1 to 60 for use in identifying a human subject who is suitable for tolerization to autoantigens in MS, preferably early MS, clinically isolated syndrome (CIS), radiologically isolated syndrome (RIS) or relapsing-remitting multiple sclerosis (RRMS).
   A peptide or polypeptide or protein as defined by any one of SEQ ID NOs: 1 to 37 is preferred, in particular any one of SEQ ID NOs: 1 to 12.
76. A method for identifying a human subject who is suitable for tolerization to autoantigens in MS, preferably early MS, CIS, RIS or RRMS, comprising measuring reactivity of the T cells and/or antibodies against the peptide and/or the combination and/or the polypeptide or protein according to any one of embodiments 1 to 34 or 36, which T cells and/or antibodies have been previously obtained from blood, CSF or other body fluid of the subject. Preferably, the method is performed *in vitro.*
   A peptide or polypeptide or protein as defined by any one of SEQ ID NOs: 1 to 37 is preferred, in particular any one of SEQ ID NOs: 1 to 12.
77. A method for identifying a human subject who is suitable for tolerization to autoantigens in MS, preferably early MS, CIS, RIS or RRMS comprising administering the peptide and/or the combination and/or the polypeptide or protein and/or the nucleic acid and/or the carrier and/or the solution and/or the pharmaceutical composition according to any one of embodiments 1 to 60. A peptide or polypeptide or protein as defined by any one of SEQ ID NOs: 1 to 37 is preferred, in particular any one of SEQ ID NOs: 1 to 12.
78. Use of the peptide and/or the combination and/or the polypeptide or protein and/or the nucleic acid and/or the carrier and/or the solution and/or the pharmaceutical composition according to any one of embodiments 1 to 60 for the manufacture of a medicament for identifying a human subject who is suitable for tolerization to autoantigens in MS, preferably early MS, CIS, RIS or RRMS. A peptide or polypeptide or protein as defined by any one of SEQ ID NOs: 1 to 37 is preferred, in particular any one of SEQ ID NOs: 1 to 12.
79. A method of manufacturing the carrier according to any one of embodiments 39 to 56 comprising providing the carrier, adding the peptide and/or the combination and/or the polypeptide or protein according to any one of embodiments 1 to 34 or 36 and a coupling reagent.
80. A method of manufacturing the chemically coupled carrier according to any one of embodiments 44 to 56 comprising providing an isolated cell, preferably a blood cell, preferably an RBC, or a population of isolated cells, preferably a population of blood cells, preferably a population of RBCs, from a human subject, adding the peptide and/or the combination and/or the polypeptide or protein according to any one of embodiments 1 to 34 or 36 and subsequently adding a coupling agent, preferably EDC.
81. A method of manufacturing the chemically coupled carrier according to any one of embodiments 49 to 52, comprising providing an isolated cell, preferably a blood cell, preferably an RBC, or a population of isolated cells, preferably a population of blood cells, preferably a population of RBCs, from a human subject, adding the combination as defined in any one of embodiments 9 to 11 and subsequently adding a coupling agent, preferably EDC.
82. The method of manufacturing the chemically coupled carrier according to any one of embodiments 46 to 56, wherein 3×10⁷ to 5×10¹⁰ RBCs/ml are provided, preferably 3×10⁸ to 3×10¹⁰ RBCs/ml, more preferably about 3×10⁹ RBCs/ml. In a preferred embodiment, an amount of 3×10¹¹ RBCs is used for the coupling reaction.
83. The method of manufacturing the chemically coupled carrier according to any one of embodiments 44 to 56, wherein each peptide is added at a concentration between 0.002 mg/ml per peptide and 2 mg/ml per peptide, in particular between 0.1 mg/ml per peptide and 1 mg/ml per peptide, for example at a concentration of about 0.002 mg/ml, 0.007 mg/ml, 0.05 mg/ml, 0.07 mg/ml, 0.125 mg/ml, 0.132 mg/ml, 0.250 mg/ml, 0.5 mg/ml, 0.7 mg/ml, 1 mg/ml, 1.5 mg/ml or 2 mg/ml per peptide. A concentration of 0.132 mg/ml per peptide is especially preferred, particularly when 12 peptides are coupled.
84. The method of manufacturing the chemically coupled carrier according to any one of embodiments 44 to 56, wherein each polypeptide or protein is added at a concentration between 0.01 mg/ml and 10 mg/ml of polypeptide or protein, more preferably between 0.5 mg/ml and 5 mg/ml. Preferably, a maximum amount of 5 different polypeptides or proteins is added.
85. The method of manufacturing the chemically coupled carrier according to any one of embodiments 44 to 56, wherein more than three, more than five, more than 10, more than 15, or more than 20 different peptides and/or polypeptides or proteins are added. In one embodiment, a maximum amount of 25 different peptides is added.
   In one embodiment, a maximum amount of five different polypeptides or proteins is added.
   In a preferred embodiment, between five and 20, preferably between five and 15 different peptides and/or polypeptide or proteins are added.
   In a particularly preferred embodiment, 12 different peptides are added.
86. The method of manufacturing the chemically coupled carrier according to any one of embodiments 44 to 56, wherein a total amount of less than 100 mg/ml, more preferably less than 50 mg/ml, even more preferably less than 20 mg/ml, even more preferably less than 15 mg/ml, in particular a maximum amount of 12 mg/ml of peptides and/or polypeptides and/or proteins is added.
87. The method of manufacturing the chemically coupled carrier according to any one of embodiments 44 to 56, wherein less than or equal to 30 mg/ml EDC are added as a coupling agent, preferably less than or equal to 25 mg/ml EDC, more preferably 0.5 to 20 mg/ml EDC, in particular 0.5 mg/ml, 1 mg/ml, 2 mg/ml, 3 mg/ml, 5 mg/ml, 10 mg/ml or 20 mg/ml EDC, more preferably 2 to 15 mg/ml, more preferably 3 to 10 mg/ml, even more preferably about 3 mg/ml EDC. In a particularly preferred embodiment, 3 mg/ml EDC are added.
88. The method of manufacturing the chemically coupled carrier according to any one of embodiments 44 to 56, wherein the coupling reaction is performed at a pH of pH 5-8.
89. The method of manufacturing the chemically coupled carrier according to any one of embodiments 44 to 56, wherein the coupling reaction is performed for about 15 min, about 30 min, about 45 min, about 60 min or about 120 min. In a particularly preferred embodiment, the coupling reaction is performed for about 60 min.
90. The method of manufacturing the chemically coupled carrier according to any one of embodiments 44 to 56, wherein the coupling reaction is performed at 15 to 25°C.
91. The peptide and/or the combination and/or the polypeptide or protein and/or the nucleic acid and/or the carrier and/or the solution and/or the pharmaceutical composition according to any one of embodiments 1 to 60 for use as medicament.
   The peptides as defined by any of SEQ ID NOs: 1 to 37, preferably SEQ ID NOs: 1 to 12, are preferred.
92. A method of controlling a quality of a cell, wherein the cell has been treated with a crosslinker and at least one peptide and/or combination and/or polypeptide or protein according to any one of embodiments 1 to 34 or 36 and wherein at least one peptide and/or combination and/or polypeptide or protein is coupled with a marker. Controlling the quality preferably comprises detecting the presence of at least one peptide and/or combination and/or polypeptide or protein on the surface of the cell.
93. Method of pretesting a human subject diagnosed with MS or a human subject at risk to develop MS using the peptide and/or combination and/or polypeptide or protein according to any one of embodiments 1 to 34 or 36. Preferably, the method is performed *in vitro.*
94. A method of treating, preventing and/or diagnosing MS comprising administering the peptide and/or the combination and/or the polypeptide or protein and/or the nucleic acid and/or the carrier and/or the solution and/or the pharmaceutical composition according to any one of embodiments 1 to 60.
95. Use of the peptide and/or the combination and/or the polypeptide or protein and/or the nucleic acid and/or the carrier and/or the solution and/or the pharmaceutical composition according to any one of embodiments 1 to 60 for the manufacture of a medicament for the treatment, prevention and/or diagnosis of MS.
96. The method or use according to embodiment 94 or 95, wherein the method of diagnosis is *in vitro, in vivo* or ex *vivo.*
97. The method or use according to any one of embodiments 94 to 96, wherein pattern I and/or pattern II MS is diagnosed.
98. A method for inducing antigen-specific tolerance to autoantigens in a human subject suffering from or at risk of developing MS comprising administering the peptide and/or the combination and/or the polypeptide or protein and/or the nucleic acid and/or the carrier and/or the solution and/or the pharmaceutical composition according to any one of embodiments 1 to 60.
99. Use of the peptide and/or the combination and/or the polypeptide or protein and/or the nucleic acid and/or the carrier and/or the solution and/or the pharmaceutical composition according to any one of embodiments 1 to 60 for the manufacture of a medicament for inducing antigen-specific tolerance to autoantigens in a human subject suffering from or at risk of developing MS.
100. A method for inducing antigen-specific tolerance to autoantigens in a human subject with early MS comprising administering the peptide and/or the combination and/or the polypeptide or protein and/or the nucleic acid and/or the carrier and/or the solution and/or the pharmaceutical composition according to any one of embodiments 1 to 60.
101. Use of the peptide and/or the combination and/or the polypeptide or protein and/or the nucleic acid and/or the carrier and/or the solution and/or the pharmaceutical composition according to any one of embodiments 1 to 60 for the manufacture of a medicament for inducing antigen-specific tolerance to autoantigens in a human subject with early MS.
102. The method or use according to any one of embodiments 94 to 95 or 98 to 101, wherein the peptide and/or the combination and/or the polypeptide or protein and/or the nucleic acid and/or the carrier and/or the solution and/or the pharmaceutical composition is administered by subcutaneous (s.c.), intracoelomic (i.c), intramuscular (i.m.), intradermal (i.d.), transdermal (t.d.) or intravenous (i.v.) administration and/or wherein the peptide and/or the combination and/or the polypeptide or protein and/or the solution and/or the pharmaceutical composition is administered by nasal, inhaled or oral administration.
103. The method or use according to any one embodiments 94 to 95 or 98 to 102, wherein the carrier is an autologous or an allogeneic cell or a population of autologous or allogeneic cells, preferably an autologous cell or a population of autologous cells, more preferably an autologous RBC or a population of autologous RBCs.
104. The method or use according to any one embodiments 94 to 95 or 98 to 103, wherein the peptide and/or the combination and/or the polypeptide or protein and/or the nucleic acid and/or the carrier and/or the solution and/or the pharmaceutical composition is injected intravenously (i.v.).
   Preferably, an RBC or a population of RBCs is coupled to the at least one peptide and/or combination and/or polypeptide or protein and/or contains the nucleic acid and is injected intravenously.
105. The method or use according to embodiment 104, wherein RBCs or a population of RBCs are/is injected intravenously and a single injection dose ranges from 1×10⁷ to 1×10¹² RBCs/kg, preferably 1×10⁹ to 1×10¹¹ RBCs/kg, more preferably 3×10⁹ to 5×10¹⁰ RBCs/kg, more preferably 3×10⁹ to 1×10¹⁰ RBCs/kg. In a preferred embodiment, an amount of 1×10¹¹ to 6×10¹¹ RBCs, preferably 2×10¹¹ to 4×10¹¹ RBCs, in particular 3×10¹¹ RBCs or 6×10¹¹ RBCs are used as a single injection dose.
106. The method or use according to embodiment 105, wherein a single dose is administered in one treatment cycle.
107. The method or use according to embodiment 105, wherein two or more doses are administered with an interval of one week to 6 months, preferably one week to 2 months.

## Claims

1. A combination comprising or consisting of the peptides TSTA3-3 (242-251) (SEQ ID NO: 1), RASGRP2 (74-91) (SEQ ID NO: 2), MBP1 (13-32) (SEQ ID NO: 3), MBP2 (83-99) (SEQ ID NO: 4), MBP3 (111-129) (SEQ ID NO: 5), MBP4 (146-170) (SEQ ID NO: 6), MOG1 (1-20) (SEQ ID NO: 7), MOG2 (35-55) (SEQ ID NO: 8), PLP1 (139-154) (SEQ ID NO:9), TSTA3-1 (51-65) (SEQ ID NO: 10), TSTA3-2 (136-150) (SEQ ID NO: 11), and TSTA3-4 (296-310) (SEQ ID NO: 12).

2. A peptide comprising or consisting of the sequence of TSTA3-3 (242-251) (SEQ ID NO: 1) and/or a peptide comprising or consisting of the sequence of RASGRP2 (74-91) (SEQ ID NO: 2) and/or a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 1 and/or SEQ ID NO: 2, respectively, wherein the peptide is soluble in a physiological solution and has a maximum length of 50 amino acids.

3. A peptide comprising or consisting of the sequence of RASGRP2 (74-91) (SEQ ID NO: 2) and/or a peptide comprising or consisting of a sequence with at least 80 %, preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 2, wherein the peptide has a maximum length of 50 amino acids.

4. A peptide consisting of the sequence of TSTA3-3 (242-251) (SEQ ID NO: 1) or RASGRP2 (74-91) (SEQ ID NO: 2).

5. A combination comprising one or more peptides according to any one of claims 2 to 4 and one or more of the following peptides:
a) a peptide comprising or consisting of the sequence of MBP1 (13-32) (SEQ ID NO: 3),
b) a peptide comprising or consisting of the sequence of MBP2 (83-99) (SEQ ID NO: 4),
c) a peptide comprising or consisting of the sequence of MBP3 (111-129) (SEQ ID NO: 5),
d) a peptide comprising or consisting of the sequence of MBP4 (146-170) (SEQ ID NO: 6),
e) a peptide comprising or consisting of the sequence of MOG1 (1-20) (SEQ ID NO: 7),
f) a peptide comprising or consisting of the sequence of MOG2 (35-55) (SEQ ID NO: 8),
g) a peptide comprising or consisting of the sequence of PLP1 (139-154) (SEQ ID NO:9),
h) a peptide comprising or consisting of the sequence of TSTA3-1 (51-65) (SEQ ID NO: 10),
i) a peptide comprising or consisting of the sequence of TSTA3-2 (136-150) (SEQ ID NO: 11),
j) a peptide comprising or consisting of the sequence of TSTA3-4 (296-310) (SEQ ID NO: 12), or
k) a peptide comprising or consisting of a sequence with at least 60 %, preferably at least 70 %, more preferably at least 80 %, more preferably at least 85 %, more preferably at least 90 %, more preferably at least 95 %, more preferably at least 96 %, more preferably at least 97 %, more preferably at least 98 %, more preferably at least 99 % identity with the sequence of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or SEQ ID NO: 12, respectively.

6. A polypeptide or protein comprising at least two peptide sequences, wherein the at least two peptide sequences are selected from the peptide and/or the combination according to any one of claims 1 to 5, optionally comprising a linker, preferably GGS, between at least two of the peptide sequences.

7. The polypeptide or protein according to claim 6, comprising or consisting of at least one of the sequences as set forth in SEQ ID NOs: 33-37.

8. The peptide and/or the combination and/or the polypeptide or protein according to any one of claims 1 to 7, wherein at least one peptide and/or polypeptide or protein is modified, preferably coupled with a marker, preferably wherein
a) the marker is biotin or a fluorescent dye, such as FAM or Cy7, preferably biotin, and/or
b) the marker, preferably the biotinylation is within the first 15, preferably within the first 10, more preferably within the first 5 amino acids from the N-terminus and/or within the last 15, preferably within the last 10, more preferably within the last 5 amino acids from the C-terminus, and/or
c) the marker is biotin and the biotinylation takes place at a cysteine, preferably at the thiol group of the cysteine, and/or
d) the marker is biotin and the biotin is coupled to the at least one peptide and/or polypeptide or protein by a polyethylene glycol (PEG) linker, and/or
e) RASGRP2 (74-91) (SEQ ID NO: 2) is biotinylated, preferably on the cysteine.

9. The peptide and/or the combination and/or the polypeptide or protein according to any one of claims 1 to 8, wherein the peptide and/or at least one peptide of the combination and/or the polypeptide or protein comprises a His Tag.

10. A nucleic acid encoding the peptide and/or the combination and/or the polypeptide or protein according to any one of claims 1 to 9.

11. A carrier comprising the peptide and/or the combination and/or the polypeptide or protein and/or the nucleic acid according to any one of claims 1 to 10.

12. The carrier according to claim 11, wherein the carrier is coupled to the peptide and/or the combination and/or the polypeptide or protein and/or contains the nucleic acid according to any one of claims 1 to 10, optionally wherein the carrier is selected from the group consisting of a cell or a population of cells, preferably a blood cell or a population of blood cells, preferably a red or white blood cell or a population of red or white blood cells, more preferably a red blood cell (RBC) or a population of RBCs, a membrane of a cell, preferably a membrane of an RBC, a protein, a lipid, a glycolipid, a bead, a nanoparticle, a virus-like-particle (VLP) and a molecule, such as a sugar molecule, and any combination thereof.

13. The carrier according to claim 11 or 12, wherein the carrier is a cell or a population of cells chemically coupled to the peptide and/or the combination and/or the polypeptide or protein according to any one of claims 1 to 9, optionally wherein the cell or population of cells is chemically coupled by 1-ethyl-3-(3-dimethylaminopropyl)-carbodiimide (ECDI/EDC).

14. The carrier according to claim 13, wherein the cell or population of cells is an RBC or a population of RBCs, chemically coupled to the combination according to claim 5.

15. A pharmaceutical composition comprising the peptide and/or the combination and/or the polypeptide or protein and/or the nucleic acid and/or the carrier according to any one of claims 1 to 14, and a pharmaceutically acceptable excipient.

16. The peptide and/or the combination and/or the polypeptide or protein and/or the nucleic acid and/or the carrier and/or the pharmaceutical composition according to any one of claims 1 to 15 for use in the treatment and/or prevention of multiple sclerosis (MS).

17. An *in vitro* method for diagnosing MS, in particular pattern I and/or pattern II MS, using the peptide and/or the combination and/or the polypeptide or protein and/or the nucleic acid and/or the carrier and/or the pharmaceutical composition according to any one of claims 1 to 15.

18. The peptide and/or the combination and/or the polypeptide or protein and/or the nucleic acid and/or the carrier and/or the pharmaceutical composition according to any one of claims 1 to 15 for use in a method for inducing antigen-specific tolerance to autoantigens in a human subject suffering from or at risk of developing MS.

19. The peptide and/or the combination and/or the polypeptide or protein and/or the nucleic acid and/or the carrier and/or the pharmaceutical composition according to any one of claims 1 to 15 for use in identifying a human subject who is suitable for tolerization to autoantigens in MS, preferably early MS, clinically isolated syndrome (CIS), radiologically isolated syndrome (RIS) or relapsing-remitting multiple sclerosis (RRMS).

20. A method of manufacturing the chemically coupled carrier according to claim 13 or 14 comprising providing an isolated cell, preferably a blood cell, preferably an RBC, or a population of isolated cells, preferably a population of blood cells, preferably a population of RBCs, from a human subject, adding the peptide and/or the combination and/or the polypeptide or protein according to any one of claims 1 to 9 and subsequently adding a coupling agent, preferably EDC.
